# EUROPEAN PATENT SPECIFICATION

(11) **EP 3 838 123 B1**
(45) Date of publication and mention of the grant of the patent: **18.11.2026**
(21) Application number: 20215319.3
(22) Date of filing: 30.04.2012
(51) Int. Cl.: G01J 3/02, A61B 5/00, A61B 3/10, G01J 3/44, G01J 3/26

(54) **METHODS AND ARRANGEMENTS FOR OBTAINING INFORMATION AND PROVIDING ANALYSIS FOR BIOLOGICAL TISSUES**
VERFAHREN UND ANORDNUNGEN ZUR GEWINNUNG VON INFORMATIONEN UND ZUR BEREITSTELLUNG VON ANALYSEN FÜR BIOLOGISCHE GEWEBE
PROCÉDÉS ET AGENCEMENTS POUR OBTENIR DES INFORMATIONS ET FOURNIR UNE ANALYSE DE TISSUS BIOLOGIQUES

(30) Priority: 29.04.2011 US 201161480885 P
(43) Date of publication of application: 23.06.2021
(62) Document number(s) of the earlier application(s) in accordance with Art. 76 EPC: 12775982.7 / 2 712 299
(73) Proprietor: The General Hospital Corporation, Boston, MA 02114 (US)
(72) Inventor: YUN, Seok-Hyun, Cambridge, MA Massachusetts 02140 (US); SCARCELLI, Giuliano, Cambridge, MA Massachusetts 02318 (US)
(74) Representative: Barker Brettell LLP

(56) References cited:
- US-A1- 2009 273 777
- US-A1- 2009 323 056
- US-A1- 2018 160 898

## Description

### CROSS-REFERENCE TO RELATED APPLICATION(S)

This application is based upon and claims the benefit of priority from U.S. Patent Application Serial No. 61/480,885, filed April 29, 2011.

### FIELD OF THE DISCLOSURE

The present disclosure relates to exemplary methods and apparatus for obtaining information and providing analysis for biological tissues, and more particularly to exemplary methods and apparatus for achieving non-invasive in vivo biomechanical and biophysical characterization, including but not limited to elasticity and viscosity, of various biological tissues, including human and animal eye via, e.g., fast optical spectroscopy and microscopy based on Brillouin light scattering.

### BACKGROUND INFORMATION

Ectasia is also one of the rare but serious adverse outcomes after LASIK (laser-assisted in situ keratomileusis) surgery. Currently about 1.5 million LASIK operations are performed annually in the U.S. As LASIK becomes increasingly popular, the incidence of post-LASIK ectasia has continued to increase. A promising therapeutic approach to corneal ectasia is increasing the stiffness of the stroma by crosslinking the naturally present collagen fibers in the cornea, a procedure known as corneal collagen crosslinking (CXL). The viscoelastic properties of the cornea are also known to affect the tonometry measurement of intraocular pressure.

As a consequence, the biomechanical properties may be an appropriate target for diagnosis and monitoring of onset and progression of cataract and presbyopia as well as corneal pathologies and treatments. For this reason, there has been a great deal of interest in measuring the mechanical properties of the lens corneal tissues for diagnosis and for monitoring of treatments. However, current techniques cannot detect such biomechanical changes in vivo in patients and animal models, seriously frustrating our efforts to develop understanding and treatment of the prevalent ocular problems.

Conventional techniques, from the traditional slit-lamp microscopy to newer imaging technologies (computer videokeratography, OCT, confocal microscopy, ultrasound, Scheimflug photography) are excellent in imaging the structure of cornea and lens but fail to provide their physiological and biomechanical information. Current clinical instruments, such as pachymetry (measuring thickness) and topography (mapping surface curvature), have been limited in screening patients at high risk of post-LASIK ectasia; patients with normal appearing corneas have developed the complication.

Several techniques have been used to characterize the mechanical properties of the cornea and lens ex vivo and in vivo. For example, comprehensive but destructive analysis has been performed by spinning cup, mechanical stretchers, stress-strain equipment or by inflation tests. Other mechanical testing methods include laser induced optical breakdown based on bubble creation and the ocular response analyze measuring corneal hysteresis on the surface without spatial information. Ultrasound is an attractive tool as it allows noninvasive methods such as elastography. Of particular note is ultrasound pulse-echo techniques and ultrasound spectroscopy, where pulsed or continuous- wave acoustic waves are launched onto the cornea, and the propagation speed and attenuation are measured to compute the viscoelastic moduli of the tissue. However, the ultrasound-based techniques have drawbacks of relatively low spatial resolution and measurement sensitivity.

Corneal ectasia refers to a bulging of the cornea, occurring when it is not strong enough mechanically to withstand the intraocular pressure. Ectasia may result from a degenerative disease called Keratoconus, affecting about 1 in 2000 people with a mean onset age of 15.4 years. (See, J. H. Krachmer et al., "Keratoconus And Related Noninflammatory Corneal Thinning Disorders," Survey of Ophthalmology, vol. 28, pp. 293-322, 1984; and J. O. Jimenez et al., "Keratoconus: Age of onset and natural history," Optometry and Vision Science, vol. 74, pp. 147-151, Mar 1997). Ectasia is also one of the rare serious adverse outcomes after LASIK surgery that results in corneal thinning. As LASIK becomes increasingly popular (about 1.5 million operations in the U.S. each year), the incidence of post-LASIK ectasia has continued to increase. Current clinical instruments, such as Pachymetry (measuring the thickness) and topography (mapping surface curvature), have been limited in screening patients at high risk of post-LASIK ectasia.

Concern about development of post-LASIK ectasia and imperfect screening tests prevents ~15% of patients seeking laser vision correction from benefiting from this procedure in the US. On the other hand, post-LASIK ectasia is reported in ~0.7% of the remaining cases in patients with normal appearing pachymetry and topography and no other risk factors. (See I. G. Pallikaris et al. , "Corneal Ectasia Induced By Laser In Situ Keratomileusis," J Cataract Refract Surg, vol. 27, pp. 1796-1802, 2001 ; P. S. Binder eta 1., "Keratoconus and corneal ectasia after LASIK," Journal of Refractive Surgery, vol. 21, pp. 749-752, Nov-Dec 2005; and Y. S. Rabinowitz, "Ectasia after laser in situ keratomileusis," Current Opinion in Ophthalmology, vol. 17, pp. 421-426, Oct 2006). This situation has led to multimillion dollar lawsuits (see, G. McDermott, "Anatomy of a lawsuit," Cataract and Refractive Surgery Today, vol. 2005, pp. 93-118, 2005), and has contributed to the decision by the FDA to reevaluate LASIK surgery safety. (See FDA, 1 "http://www.fda.gov/NewsEvents/Newsroom/PressAnnouncements/2009/ucm186858.htm." 2009). There is a growing consensus that the mechanical properties of the cornea, e.g. elastic modulus or stiffness, given their connection to the pathophysiology of the condition, are highly suited to early identify patients at risk for developing ectasia. (See Y. Rabinowitz, "Ectasia after laser in situ keratomileusis," Current opinion in ophthalmology, vol. 17, pp. 421-427, 2006; and J. W. Ruberti et al., "Corneal Biomechanics and Biomaterials," in Annual Review of Biomedical Engineering, Vol 13. vol. 13, M. L. D. J. S. G. M. L. Yarmush, Ed., 2011, pp. 269-295).

A further therapeutic approach to corneal ectasia can increase the stiffness of the stroma by crosslinking the naturally present collagen fibers in the cornea - a procedure known as corneal collagen crosslinking (CXL). For this procedure, monitoring the mechanical properties of the ocular tissue before, during and after, can assist with optimizing the outcome and success of the intervention.

Measuring the mechanical properties of the cornea can also be beneficial for glaucoma screening. Glaucoma is the second highest cause of blindness in the world. About 2.2 million Americans have glaucoma and half of them are not aware of their condition. Left untreated, glaucoma leads to blindness; if diagnosed early and managed, 90% of the patients avoid blindness. Elevated intraocular pressure (IOP) is a known risk factor for glaucoma and is currently used as major screening parameter. To evaluate IOP, applanation tonometry measures the external pressure required to applanate the cornea. However, applanation tonometry can assume the cornea to be thin, spherical and elastic; therefore, pressure readings can be biased by actual corneal thickness, curvature and stiffness. While pachymetry and tomography can measure corneal thickness and curvature, stiffness is not currently accounted for. Modeling studies show that lack of stiffness information introduces an uncertainty in IOP estimation of 2 to 3 mmHg (enough to misclassify the risk category of patients). The effect can be more pronounced in patients who underwent refractive surgery or crosslinking. Without corneal stiffness information, IOP measurements are therefore unreliable.

As a consequence, the biomechanical properties may be an appropriate target for diagnosis and monitoring of onset and progression of cataract and presbyopia as well as corneal pathologies and treatments. For this reason, there has been a great deal of interest in measuring the mechanical properties of the lens corneal tissues for diagnosis and for monitoring of treatments. However, prior techniques may not be able to detect such biomechanical changes *in vivo,* e.g., in patients and animal models, seriously frustrating efforts to develop understanding and treatment of the prevalent ocular problems.

Several techniques have been developed to characterize the mechanical properties of the cornea and lens ex vivo and in vivo. For example, comprehensive but destructive analysis has been performed by spinning cup, mechanical stretchers, stress-strain equipment or by inflation tests. Other prior mechanical testing methods can include laser induced optical breakdown based on bubble creation and the ocular response analyze measuring corneal hysteresis on the surface without spatial information. Ultrasound can be a possible approach, since it facilitates noninvasive methods such as elastography. For example, ultrasound pulse-echo techniques and ultrasound spectroscopy can be used, where pulsed or continuous-wave acoustic waves are launched onto the cornea, and the propagation speed and attenuation are measured to compute the viscoelastic moduli of the tissue. However, the ultrasound-based techniques have certain drawbacks, such as, e.g., a relatively low spatial resolution and a particular measurement sensitivity.

Brillouin scattering results from the interaction of an incident optical wave with hypersonic acoustic phonons inside the medium under test. In Spontaneous Brillouin scattering the acoustic phonons are inherently present in the material due to thermally- induced density fluctuations. However, the Brillouin process can be enhanced or forced by using multiple optical pump waves with frequencies separated by those of the acoustic phonons in the medium. The technique that analyzes the feature of Brillouin scattering signal is known as Brillouin spectroscopy. Various techniques to detect the Brillouin signal have been known in the art and are widely applied in physics, material science, and mechanical engineering.

The magnitude and frequency (spectrum) of the Brillouin scattered light are determined by hypersonic acoustic phonons that are inside the material, the latter being closely related to the mechanical properties of the medium, such as elastic and viscous modulus. These mechanical properties therefore can be measured by Brillouin spectroscopy. In addition, Brillouin spectrum can also be dependent on temperature, pressure, density and refractive index; hence, under specific conditions the analysis of Brillouin spectrum can be used to measure these quantities.

Brillouin spectroscopy of the cornea and lens has been performed using tissues ex *vivo.* (See Y. S. Rabinowitz, "Ectasia after laser in situ keratomileusis," Current Opinion in Ophthalmology, vol. 17, pp. 421-426, Oct 2006). However, the significant potential of using Brillouin scattering for ocular biomechanics has not been fully exploited and *in vivo* measurement has not been demonstrated. From a technological point of view, the long acquisition times required by the spectral analysis have limited the technique to point spectroscopy while the limited extinction of spectral instruments has made it difficult to discriminate Brillouin scattering signal from the elastic scattering or the scattering from various optical components.

Although Brillouin spectroscopy has been used for material characterization, structural health monitoring, and environmental sensing, the combined requirements of spectral resolution (10⁻³ nm) and spectral extinction (to suppress strong elastic scattering) forced using slow sequential spectrometers, scanning Fabry-Perot (FP), which acquire a single Brillouin spectrum likely in about a number of minutes to hours. (See B. Culshaw et al., "Smart structures and applications in civil engineering," Proc. of the IEEE, vol. 84, pp. 78-86, 1996; G. D. Hickman et al., "Aircraft laser sensing of sound velocity in water: Brillouin scattering, " Rem. Sens. Env., vol. 36, p. 165, 1991; and J. R. Sandercock, "Some recent developments in Brillouin scattering," Rca Review, vol. 36, pp. 89-107, 1975). This can limit the technology to point-sample analysis.

In the past, a spectrometer has been provided which can use a modified FP etalon called a virtually-imaged-phased-array (VIPA), that facilitated parallel detection. (See G. Scarcelli and S. H. Yun, "Brillouin Confocal Microscopy for three-dimensional mechanical imaging," Nature Photonics, vol. 2, pp. 39-43, 2008; and M. Shirasaki, "Large angular dispersion by a virtually imaged phased array and its application to a wavelength demultiplexer," Opt. Lett., vol. 21, pp. 366-368, Mar 1996). This greatly reduced the acquisition time to about 1-10 seconds per spectrum in transparent materials. (See G. Scarcelli and S. H. Yun, "Brillouin Confocal Microscopy for three-dimensional mechanical imaging," Nature Photonics, vol. 2, pp. 39-43, 2008) This positive result, however, was still not sufficient to achieve an appropriate Brillouin imaging in tissue *in vivo,* or rapid monitoring of tissue mechanical properties.

Accordingly, there may be a need to overcome at least some of the issues and or deficiencies described herein above.
US 2009/0273777 A1 discloses an apparatus and method to measure and filter the spectrum of electro-magnetic radiation using multiple dispersive elements, such as diffraction gratings or VIPA etalons, concatenated in a cross-axis orthogonal arrangement.

### OBJECTS AND SUMMARY OF THE INVENTION

To address and/or overcome the above-described problems and/or deficiencies, exemplary embodiments of systems, arrangements and processes can be provided that are capable of obtaining information and providing analysis for biological tissues. For example, exemplary methods and apparatus can achieve non-invasive in vivo biomechanical and biophysical characterization, including but not limited to elasticity and viscosity, of various biological tissues, including human and animal eye via, e.g., fast optical spectroscopy and microscopy based on Brillouin light scattering. In certain exemplary embodiments of the present disclosure, apparatus and methods can be provided to obtain non-invasively the information about intrinsic biomechanical properties of biological tissue (e.g., including but not limited to human and other animal eyes), which can be utilized in a wide range of applications in clinical diagnosis of, e.g., ocular problems, research, development of treatments using animal models, etc..
According to the invention there is provide a system and method for rapid spectroscopic analysis of a biological tissue, as defined by the appended claims.

According to certain exemplary embodiments of the present disclosure, it is possible to perform a spectral analysis to facilitate an in vivo tissue characterization including, e.g., a) sub-second Brillouin spectrum acquisition, b) higher than 60 dB rejection of elastic scattering, and c) sub-GHz elasticity sensitivity. For example, by combining an exemplary spectrometer having certain exemplary characteristics to achieve such exemplary results with a confocal microscope, it can be possible to characterize ocular tissue, lens and cornea in vivo, e.g., in mice and human patients, as well as in and for skin tissue in animals and human. In addition, using such exemplary embodiments, "elasticity imaging" can be accomplished with a high spatial resolution. According to the exemplary embodiments of the present disclosure, there is a benefit over ultrasound techniques, as significantly higher resolution and sensitivity can be obtained. Indeed, it is possible to probe the elasticity of the tissues non-invasively with a microscopic resolution.

Through elasticity imaging, it is possible to utilized certain exemplary protocols, e.g., to extract quantitative biomechanical parameters that can characterize the biophysical/biomechanical state of the biological tissue under consideration. In this manner, health status, risk to develop conditions and outcome of the exemplary procedures can be assessed beyond structural information. Therefore, according to the exemplary embodiments of the technique which uses Brillouin principles, it is possible to enhance management of a variety of ocular problems by, e.g., providing currently inaccessible, biophysical and bio-structural data of the lens and cornea.

According to an exemplary embodiment of the present disclosure which utilizes Brillouin spectroscopy and imaging, it is possible to overcome many of the current state of the art technology limitations and/or deficiencies, such as slit lamp, pachymetry, and topography. Other imaging devices, such as the Pentacam (rotating Schiemflug imaging), generally provide only static information about the lens and cornea and likely do not provide biophysical dynamic information about the cornea or lens. Likewise, the Ocular Analyzer (corneal hysteresis) generally provides only limited information about the cornea regarding its elasticity, and no imaging information or data about the lens. In certain exemplary embodiments of the present disclosure, it is possible to provide quantitative values, and a high quality image of biophysical properties of lens and cornea. This can facilitate in screening of high at-risk patients of corneal ectasia and diagnosing diseases such as keratoconus and cataracts.

According to further exemplary embodiments of the present disclosure, it is possible to utilize to analyze ocular tissue elasticity behavior versus frequency of induced stress and validating Brillouin microscopy against high-end mechanical tests. In addition, an exemplary in vivo biomechanical analysis can be tested against the known natural stiffening that occurs in aging small animals to demonstrate the ability of this technology of providing information of clinical relevance.

In one exemplary embodiment of the present disclosure, it is possible to utilize a spectroscopic analysis that is performed on a high-speed basis, with high spectral resolution and high extinction. The use of such exemplary technique can be done with an ophthalmic slit lamp to facilitate a mechanical image formation. For example, a pump beam can be scanned over a sample (e.g., a biological tissue, such as an eye) through an objective lens, and Brillouin-shifted optical waves are detected to characterize the Brillouin spectrum. The measured spectral features of the Brillouin signal can represent the contrast mechanism for imaging; an image can be obtained by use of an exemplary lookup table and/or an appropriate processing computational routine. Microscopically-resolved three-dimensional images of biomechanical properties of cornea, crystalline lens, aqueous and vitreous humor of the human eye, as well as other tissues can be obtained by using, e.g., a high numerical-aperture objective lens and confocal detection.

According to another exemplary embodiment of the present disclosure, a biomechanical analysis of the human eye can be combined with Raman spectroscopy, fluorescence and auto-fluorescence microscopy, as well as reflectance microscopy. The exemplary co-registration of multiple contrast mechanisms can facilitate, e.g., a complete or near-complete characterization of the biophysical properties, such as structure, elasticity, chemical composition, functional abilities of the human eye in three dimensions with micron-scale resolution, etc.

According to still further exemplary embodiments of the present disclosure, enhanced Brillouin scattering can be facilitated using multiple pump beams, auxiliary ultrasound, or contrast-enhancing nanoparticles. Various pump and probe techniques, heterodyne or spectrometer-based detection techniques can be used. Since the Brillouin shift in human tissues can range typically from about 10 MHz to 10 GHz, the direct electrical detection of the acoustic wave may also be possible.

According to particular exemplary embodiments of the present disclosure, arrangements and methods can be provided for determining information associated with at least one section of at least one biological tissue. For example, it is possible (e.g., using at least one first arrangement) to provide at least one first electro-magnetic radiation to the section(s) of the biological tissue(s) in vivo so as to interact with at least one acoustic wave in the biological tissue(s). At least one second electro-magnetic radiation can be produced based on the interaction. With at least one second arrangement, at least one portion of the second electro-magnetic radiation(s) can be received, and the information associated with the section(s) of the biological tissue(s) can be determined based on the portion of the second electromagnetic radiation(s).

For example, the section(s) of the biological tissue(s) can include an ocular tissue of an eye, which can be cornea, aqueous humor, crystalline lens, vitreous humor, or retina in the eye *in vivo.* The first arrangement(s) can include a radiation emitting source arrangement which can be configured to provide the first electro-magnetic radiation(s), the first radiation(s) can have a wavelength in the range of about 450-1350 nm with a spectral width less than 1 GHz. The second arrangement(s) can include a spectrally-resolving arrangement which can be configured to measure at least one spectral characteristic of the portion(s) of the second electromagnetic radiation. The second arrangement(s) can obtain the information associated with the section(s) of the biological tissue based on the spectral characteristic(s).

The spectrally-resolving arrangement can include a spectrometer that is configured to disperse the spectrum of the second electromagnetic radiation(s). The spectrometer can have a spectral extinction efficiency greater than about 60 dB. The spectrometer can include at least one virtually imaged phased array (VIP A) etalon that can be configured to disperse the spectrum of the second electromagnetic radiation(s). The spectrometer can include an apodization filter and/or an apodized etalon that is configured to provide a spectral extinction efficiency greater than about 60 dB. 10. The spectral characteristics) can be at least one frequency difference between the first electro-magnetic radiation(s) and the portion(s) of the second electro-magnetic radiation(s) The frequency difference(s) can be associated with a propagation speed of the at least one acoustic wave, and the frequency difference can be in the range of about 2 GHz to 20 GHz. The information associated with the section of the biological tissue can be at least one image or a spatially- resolved map of the at section(s) based on at least one parameter associated with the frequency difference. The parameter(s) can include a visco-elastic modulus. The spectral characteristic can be at least one spectral line width of the portion(s) of the second electromagnetic radiation. The spectral line width can be associated with a propagation attenuation of the acoustic wave(s), and the frequency line width can be in the range of about 0.3 GHz to 3 GHz.

In another exemplary embodiment of the present disclosure, the information associated with the section(s) can be maximum, average, and/or rate of variation of at least one parameter related to the frequency difference over the at least section. At least one third arrangement can be provided that is configured to facilitate positioning the biological tissue(s) with respect to the first electro-magnetic radiation(s). The third arrangement(s) can include an imaging arrangement configured to measure at least one position of the first electro-magnetic radiation(s) with respect to the biological tissue(s). The second arrangement(s) can determine the information based on the position(s). At least one fourth arrangement can be provided which is configured to receive the first electromagnetic radiation(s), and generate a fourth radiation based on an interaction of the first electromagnetic radiation(s) with an acoustic wave in a reference material. A spectrum of the fourth radiation can be used to determine the information by the second arrangement(s).

In still another exemplary embodiment of the present disclosure, the portion(s) of the second electromagnetic radiation(s) can be provided by Brillouin scattering using the first electro-magnetic radiation in the biological tissue. The first arrangement(s) can be further configured to provide the first electromagnetic radiation to at least one segment which is at least one point, at least one line and/or an area on or within the biological tissue. The second arrangement(s) can be configured to determine the information from the segment(s) in less than 0.4 seconds (or in less than 1 second and or at least 0.1 seconds); where the total optical power of the first electro-magnetic radiation is less than 1 mW. The first arrangement(s) can be further configured to provide the first electromagnetic radiation(s) to a further segment, and the second arrangement(s) can be further configured to generate at least one image for the segment and the further segment. The first arrangement(s) can cause a movement of the first electromagnetic radiation(s) from the segment to the further segment in a transverse direction and/or a longitudinal direction with respect to the section(s).

For example, the information can include (i) a biomechanical property, (ii) a stiffness, and/or (iii) a cross-linking of the biological tissue, (iv) a stiffness, (v) an accommodation power, (vi) a presbyopia, or (vii) a cataract of the crystalline lens, (viii) stiffness, (ix) a keratoconus, or (x) a risk of ectasia for a refractive surgery, (xi) collagen crosslinking of the cornea, and/or (xii) intraocular pressure of the eye. At least fourth arrangement can be provided that is configured to produce at least one image associated with the characteristic of the biological tissue. The point can include a plurality of points, and the second arrangement(s) can determine the information from the plurality of points. The second arrangement(s) can determine the information from the plurality of points in less than 0.4 seconds.

In still another exemplary embodiment, apparatus and method can be provided according to the present disclosure. For example, using at least one first arrangement, it is possible to provide at least one first electro-magnetic radiation to at least one portion of at least one sample so as to generate at least one second electro-magnetic radiation with multiple spectral peaks. Further, using at least one second arrangement, it is possible to receive at least one portion of the second electro-magnetic radiation(s) so as to simultaneously acquire spectrum of the portion of the second electromagnetic radiation(s) having the multiple spectral peaks in a range of about 2 GHz to 200 THz.

The portion(s) of the second electro-magnetic radiation(s) having at least one of the spectral peaks can be associated with Brillouin scattering, Raman scattering, fluorescence and/or luminescence, and the portion(s) of the second electro-magnetic radiation(s) having another one of the spectral peaks can be associated with at least different one of Brillouin scattering, Raman scattering, fluorescence and/or luminescence. The sample can be a biological tissue. The first electro-magnetic radiation(s) can be directed to the biological tissue *in vivo.*

These and other objects, features and advantages of the present invention will become apparent upon reading the following detailed description of embodiments of the disclosure, when taken in conjunction with the appended claims.

### BRIEF DESCRIPTION OF THE DRAWINGS

Further objects, features and advantages of the present disclosure will become apparent from the following detailed description taken in conjunction with the accompanying drawings showing illustrative embodiments of the present disclosure, in which:
Figure 1A is a diagram of a system and/or an apparatus according to an exemplary an exemplary embodiment of the present disclosure;
Figure 1B is a diagram of the system/apparatus according to another exemplary embodiment of the present disclosure;
Figure 2A is a graph of an exemplary frequency-dependent longitudinal modulus of typical viscoelastic biological samples;
Figures 2B and 2C are graphs of exemplary measurement results between a conventional Instron stress-strain test (x-axis) and an exemplary embodiments of Brillouin measurement;
Figure 3 is a combinational diagram illustrating exemplary principles of cross-axis cascading according to exemplary embodiments of the present disclosure;
Figure 4A is a configuration of a spectrometer using apodized VIPA etalons according to exemplary embodiments of the present disclosure;
Figure 4B is an enlarged view of design and output beam profiles of an apodization filter schemes in the spectrometer provided in an exemplary configuration of Figure 4A according to exemplary embodiments of the present disclosure;
Figure 4C is graph of an exemplary Brillouin shift of an epidermal layer in hydrated conditions according to exemplary embodiments of the present disclosure;
Figure 5A is an illustration of various examples illustrating how a focus is scanned over an eye to obtain the biomechanical information at multiple locations in ocular tissues, and thereby to obtain Brillouin images, according to exemplary embodiments of the present disclosure;
Figure 5B is an illustration of various exemplary imaging patterns to optimize the imaging speed and sampling resolution according to exemplary embodiments of the present disclosure;
Figure 5C is a schematic diagram and an image illustrating an exemplary principle of a single- VIPA spectrometer configured to interrogate multiple spatial locations in the eye simultaneously; according to exemplary embodiments of the present disclosure;
Figure 5D is a schematic diagram and an image illustrating an exemplary principle of a single- VIPA spectrometer that uses a line-focused probe beam according to further exemplary embodiments of the present disclosure;
Figure 6A is an exemplary illustration and animal images according to exemplary embodiments of the present disclosure;
Figure 6B is a set of exemplary images illustrating experimental data of Brillouin spectra obtained with a two-state VIPA spectrometer at four different locations in a murine lens *in vivo*;
Figure 6C is a graph of an exemplary Brillouin frequency shift measured as a function of depth in the region spanning from the aqueous humor through a lens to the vitreous, according to exemplary embodiments of the present disclosure;
Figure 6D is an illustration of an axial profile of a width of the Brillouin spectrum over depth according to exemplary embodiments of the present disclosure;
Figure 7A is a cross-sectional exemplary Brillouin image of a bovine cornea obtained using the system and method according to exemplary embodiments of the present disclosure;
Figure 7B is an en-face exemplary Brillouin image of the bovine cornea according to exemplary embodiments of the present disclosure;
Figure 7C is a graph of an exemplary axial profile of the Brillouin shift in the bovine cornea obtained using the system and method according to exemplary embodiments of the present disclosure;
Figure 8A is a set of cross-sectional exemplary Brillouin images of a corneal tissue before and after cornea cross-linking obtained using the system and method according to exemplary embodiments of the present disclosure;
Figure 8B is a graph indicating a marked difference between normal and crosslinked cornea tissues in terms of the axial slope of Brillouin shift in the stroma;
Figure 8C is a graph indicating a significant difference in a space-averaged Brillouin modulus between the normal and cross-linked corneal tissues;
Figures 9A-9C are graphs of exemplary profiles of the longitudinal elastic modulus of two fresh crystalline lenses obtained with Brillouin microscopy according to exemplary embodiments of the present disclosure;
Figures 9D-G are graphs of age versus the longitudinal modulus in view of the graphs shown in Figures 9A-9C;

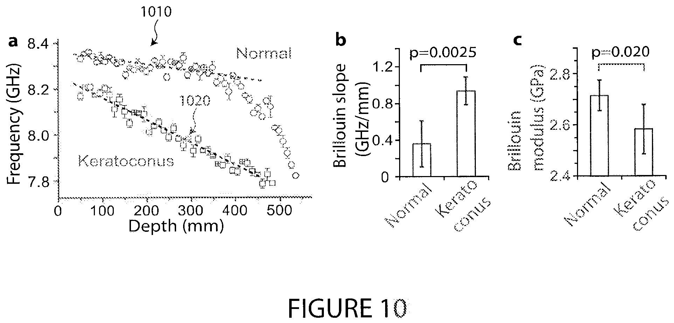

Figure 10 is a set of graphs associated with exemplary profiles of corneas classified as normal and keratoconus, illustrating them to be dramatically different;
Figures 11A-11D is a set of graphs illustrating exemplary measured axial profile obtained from a left eye of a subject from both cornea using the system and method according to exemplary embodiments of the present disclosure;
Figure 12A is a graph of a representative Brillouin spectrum from porcine epidermis obtained using the system and method according to exemplary embodiments of the present disclosure;
Figure 12B is a graph of exemplary results of Brillouin spectroscopy which indicates a higher frequency shift for the dermal layer with respect to the epidermis according to exemplary embodiments of the present disclosure; and

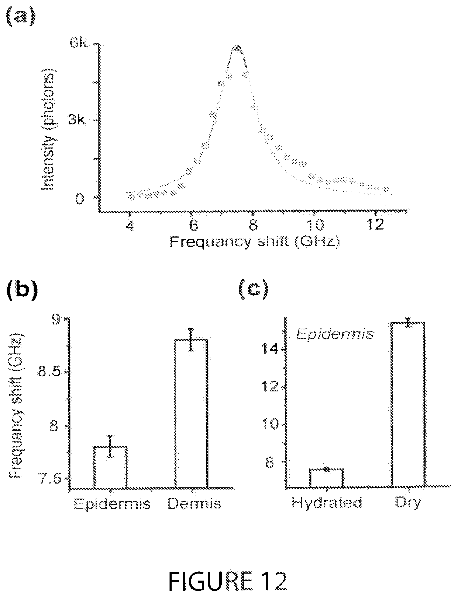

Figure 12B is a graph of exemplary results of Brillouin spectroscopy which indicates a higher frequency shift for the dry epidermis according to exemplary embodiments of the present disclosure.

Throughout the figures, the same reference numerals and characters, unless otherwise stated, are used to denote like features, elements, components or portions of the illustrated embodiments. Moreover, while the subject invention will now be described in detail with reference to the figures, it is done so in connection with the illustrative embodiments. It is intended that changes and modifications can be made to the described embodiments without departing from the true scope of the invention as defined by the appended claims.

### DETAILED DESCRIPTION OF EXEMPLARY EMBODIMENTS

According to an exemplary embodiment of the system, method and apparatus according to the present disclosure, it is possible to perform a Brillouin microscopy in ocular tissue in vivo, which can be valuable in ocular biomechanical characterization in diagnosing and treating ocular problems, as well as developing novel drugs or treatments.

There are four anatomical sites in the eye: For example, the cornea is a thin (e.g., less than 1 mm) tissue composed by different layers of varying mechanical strength. The aqueous humor is a liquid with similar properties to water that fills the anterior chamber of the eye. The crystalline lens is a double-convex sphere composed by many layers of different index of refraction, density and stiffness. The vitreous humor is the viscous transparent liquid that fills the posterior chamber of the eye.

Brillouin light scattering in a tissue or any other medium usually arises due to the interaction between an incident light and acoustic waves within the matter. For example, a probe light having a frequency v and a wavelength λ can be used, which may be provided to a sample. In a Spontaneous Brillouin process, the acoustic waves or acoustic phonons are naturally present due to thermal fluctuations. Such fluctuations propagate through the medium in the form of acoustic waves. These acoustic waves can generate periodic modulations of the refractive index. Brillouin scattering can be generated by at least one or many acoustic waves or acoustic phonons, which form phase-matched index modulation.

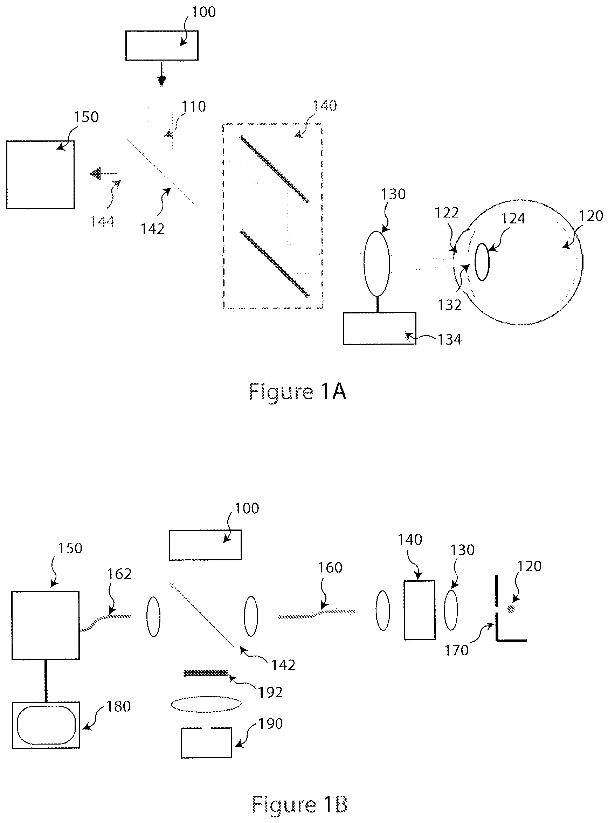

Figure 1A illustrates a diagram of a system/apparatus according to an exemplary embodiment of the present disclosure. In this exemplary system/apparatus, a first arrangement 100 can provide a first electromagnetic radiation 1 10, which can be delivered to an eye 120. One exemplary form of the electromagnetic radiation 1 10 can be light in the visible or near infrared range. The first arrangement 100 can include a radiation emitting (e.g., light) source, which can be a single-frequency laser, a filtered Mercury lamp, or other types of light emitters known in the art. The source can have a wavelength between, e.g., about 530 nm and 1350 nm, although other wavelengths that are known to be safe for use in the eye can be used. The line width of the radiation 1 10 can be typically less than about 1 GHz or more preferably less than about 100 MHz, although other light sources with broader line width or multiple spectral lines can be used in conjunction with appropriate arrangements. The radiation source can utilize an optical arrangement to deliver more than one frequency lines in order to enhance Brillouin scattered signal. The scattered radiation (e.g., light) from the sample can include multiple frequency components originated from simple elastic scattering as well as Brillouin scattering. To increase spectral purity and line width of the laser light, a single longitudinal mode lasers with high side-mode suppression ratios can be used, or optical devices to select a single frequency can also be utilized. Such devices can be interferometers, etalons, such as VIPA or Fabry Perot, gas chambers or other narrow bandpass filters, etc.

The electromagnetic radiation 110 can be directed to the eye 120 to probe various portions of ocular tissues, including but not limited to, cornea 122 and a crystalline lens 124. For example, an imaging lens 130 can be used to focus the electromagnetic radiation 110 onto a small spot. The imaging lens 130 can be a spherical convex lens, aspheric lens, objective lens, theta lens, or cylindrical lens for line focusing.

To scan the axial position of the focus within the ocular tissues, the imagining lens 130 can be mounted on a translation stage 134. Alternatively or in addition, a tunable element that can change a divergence of the probe radiation can be employed. To scan the transverse position of the focus, a one- or two-axis beam scanner 140 can be employed. The exemplary scanner 140 can include a galvanometer-mounted mirror, MEMS mirror, translation stages, spatial light modulator, etc..

An acousto-optic interaction in the tissue can give rise to light/radiation scattering, thereby generating at least one second electromagnetic radiation. Several mechanisms for light radiation scattering are known in the art, including Rayleigh and Mie scattering, Raman scattering, and Brillouin scattering. While biological tissues support these scattering mechanisms, Brillouin scattering is directly associated with the acoustic waves in the medium. A portion of such one or more second electromagnetic radiations can be collected by the imaging lens 130.

The exemplary system of Figure 1A can utilize a beam splitter 142 to reflect and transmit the first and second electromagnetic radiations. The beam splitter 142 can have, e.g., an equal 50/50 splitting ratio or unequal splitting ratios for optimization of the efficiencies of signal generation and collection. The beam splitter 142 can be a neutral splitter with broad spectral bandwidth or a dichroic splitter based on multilayer coating, interference, or diffraction. The portion of the second electromagnetic radiation 144 can be transmitted to a second arrangement 1 0, which can be configured to receive the at least one portion 144 of such one or more second electro-magnetic radiations 144.

Figure IB illustrates a diagram of the exemplary system/apparatus according to another exemplary embodiment of the present disclosure. For example, the exemplary system/arrangement of Figure IB can use an optical fiber 160 between the first arrangement 100 and the beam scanner 140. Another optical fiber 162 can also be used to deliver the Brillouin scattering light to the second arrangement 150. The optical fiber 160 in the sample arm can be a single-mode fiber, although multi-mode, few-mode, or double-clad fibers can also be used. For example, the optical fiber 162 in the detection arm can be a single-mode or few-mode fiber. The optical fiber 162 can serve as a confocal pinhole, facilitating the selective collection of essentially only the portion of the second electromagnetic radiation generated from the focus of the probe light in the sample. This exemplary confocal detection can facilitate the spatially resolved Brillouin measurement with three-dimensional resolution. The confocal detection is well known in the art. Instead of the optical fiber 162, a spatial filter, such as employing a pinhole, may be used. It is possible to minimize and/or reduce optical reflections at various air-glass or air-tissue interfaces along the beam paths or prevent the reflected light from entering the second arrangement 150 as much as possible.

The beam scanning apparatus can be mounted on an ophthalmic slit lamp instrument, and a very low power auxiliary laser beam can be used so that the operator can view the eye and aim the probe laser at the target location in the eye. For small animal studies, another exemplary embodiment of the present disclosure can be used without the slit lamp. If not mounted on the slit lamp, the exemplary system can further comprise a third arrangement , which can be configured to facilitate positioning the eye 120 with respect to the first electro-magnetic radiation(s), and/or the probe light. Preferably, the third arrangement can include at least one of the following features: a forehead rest, a chin rest, an eye fixation beam, and or a bite bar to maintain repeatable position over long-term analysis, etc. For example, the human interface 180 can employ a camera to measure at least one position of the at least one first electro-magnetic radiation with respect to the at least one ocular tissue. This type of beam guiding arrangement can facilitate aiming the probe beam and provide the position information of the focus, which can be used in making of Brillouin images or the spatial map of the biomechanical properties of the ocular tissue.

Several exemplary configurations for illumination collection of light from ocular tissue can be employed. In an epi-detection configuration, the interacting probe and Brillouin-scattered lights travel in the nearly opposite directions. Alternatively or in addition, a dual- axis configuration can be employed, where the probe and scattered light can form a finite angle. A theta lens or a microscope objective lens with a long working distance can be used for the exemplary Brillouin detection. For coarse resolutions, a collimated pump beam with a relatively small beam diameter can be used. For three dimensional resolutions, the pump/probe beams can be focused into the eye by the use of objective lenses.

In this respect, cornea, lens, aqueous and vitreous humors do not absorb light in the visible and near infrared wavelengths, while the retina is sensitive to such radiation. Moreover, the eye itself can be considered as an optical system that may tend to focus parallel beams onto the retina. As a consequence, to increase and/or maximize the input power, and thus Brillouin signal, while avoiding eye damage, the focusing strategy should be carefully considered. When possible, it may be preferable to focus the light onto the cornea and on to the lens with high numerical aperture objectives because their fast defocusing will minimize the light intensity delivered to the retina.

The selection of the focusing/collecting strategy and the objective lens generally takes into account other factors. For example, in the epi-detection configuration, the backward-propagating Brillouin light can be detected by the same objective lens used for illumination. In dual axis configuration,, e.g., illumination and Brillouin light can be either detected by the same lens (small angles) or detected by two different lenses (larger angles, longer working distance). Epi-detection can achieve the maximum collectable signal at given illumination power. In particular, the advantage in collection efficiency can be calculated to be, e.g., 2*sin(29)/3*NA, where 2Θ is the dual-axis angle and NA is the numerical aperture of the objective lens. Moreover, epi-detection configuration can facilitate the beam scanning over the sample. On the other hand, the dual axis configuration can increase and/or maximize axial resolution at given NA. In addition, a dual axis configuration greatly reduces back-reflections from optical components, thus relaxing the requirements on extinction from the spectrometer.

In terms of objective lenses, exemplary lenses with low numerical aperture (NA) result in a low transverse resolution, but the longitudinal interaction length is long and well defined. Objective lenses with high NA, generally give better transverse and axial resolution. Since the interaction can be made over a large solid angle, the line width of the scattered light broadens affecting the strength of Brillouin signal and the accuracy of the spectral analysis.

Exemplary confocal techniques can be used to enhance depth sectioning. For the same or similar purpose, a single mode fiber can be used as confocal pinhole. Acting as tight spatial mode filter, the fiber can facilitate a strict confocal imaging, thereby minimizing any stray or spurious unwanted radiation. On the other hand, to increase the signal level, a multi-mode collection can be employed. For example, a double-clad fiber with a large inner cladding can collect light with the collection efficiency about 50-200 times higher than that of a single mode fiber currently used in our prototype. Double-clad fibers generally enhance the signals in fluorescence endoscopy and white-light scattering. This exemplary approach can reduce confocal sectioning, therefore the optimal balance between improved SNR and confocal sectioning can be found.

According to one exemplary embodiment of the present disclosure, the second arrangement ISO can employ at least one spectral analysis unit, such as a spectrometer, a monochromator, fixed or scanning spectral filters, or other devices known in the art. The second arrangement 150 can be configured to measure various properties of the second electromagnetic radiation 144, including but not limited to, the center frequency and width of its spectrum, as well as the intensity and polarization of the electrical field. In particular, the frequency difference between the first electromagnetic radiation(s) 1 10 entering the tissues and the portion of the second electromagnetic radiation(s) 144, which includes the Brillouin scattered light, can be of importance.

The exemplary system/apparatus can further comprises a fourth arrangement 180 configured to display the information associated with the at least one portion of the ocular tissue in the eye in vivo. The displayed information may include but is not limited to the Brillouin frequency shifts, Brillouin line width, Brillouin images, and the hypersonic viscoelastic moduli, as well as parameters, such as the mean values or slopes, calculated from the Brillouin images or the spatial maps of the viscoelastic properties.

The exemplary system/apparatus can further utilize a fifth arrangement 190 to provide at least one frequency reference. For example, the fifth arrangement 190 can be configured to receive at least one portion of the first electromagnetic radiation through the beam splitter 142, and reemit Brillouin scattered light with at least one, and possibly multiple, spectral peak(s). For example, the frequency reference 190 can include at least one reference material, solids or liquids, with known Brillouin frequency shifts. Alternatively or in addition, the frequency reference 190 can be a light source emitting an electromagnetic radiation at a wavelength locked to the wavelength of the probe light source 100. In both case, the electromagnetic radiation from the frequency reference 190 is directed to the second arrangement 150. An optical switch 192 can be employed to gate the intensity of the electromagnetic radiation. The reference frequency can assist with calibrating the spectral analysis unit in the second arrangement 150, facilitating the spectral analysis.

In another exemplary embodiment, additional arms can be added to the microscope to measure the Brillouin scattering signal from known materials. Having two additional reference materials can be sufficient to have a constantly calibrated instrument that can automatically correct for small variation of inside the spectrometer due to temperature instabilities or mechanical drifts.

The frequency shift VB of the Brillouin scattered light with respect to the probe light 110 can be given by ${v}_{B} = \pm \frac{2 nV}{λ} sin \left(\frac{θ}{2}\right)$ (3) Where *n* is the local refractive index in the interrogated tissue, *V* is the speed of the acoustic wave in the sample, and *θ* is the scattering angle, i.e. the angle between the incident and the scattered light, such as in the dual-axis geometry. In an epi-backward detection configuration, θ = π can be a reasonably good approximation. In typical soft tissues, the speed of the acoustic wave can range from about 1000 to 3000 m/s, and the Brillouin frequency shifts can typically be between 2 and 20 GHz, depending on the wavelength.

The intrinsic spectral width or line width of the Brillouin scattered light can be given by: $Δ {v}_{B} = \frac{αV}{π} ,$ (4) where *α* is the attenuation coefficient of the acoustic wave in the sample.

The longitudinal complex elastic modulus, *M* = *M'+iM",* where the real part M' refers to the elastic modulus and the imaginary part M" is the viscous modulus is given by: $M ′ = {ρV}^{2} ;$ (5) $M " = 2 {ρV}^{3} α / {v}_{B} .$ (6)

Although the physical mechanism of Brillouin scattering can be known, the meaning of Brillouin signatures has not been studied in the context of its biomechanical significance of complex biological material. The biomechanical information extracted from Brillouin spectroscopy may not be the familiar stiffness that is felt upon palpation and is not the standard elastic modulus that can be obtained from a macroscopic stress-strain test. The Brillouin viscoelastic moduli can be defined in Equations (5) and (6) represent the tissue properties at the hypersonic GHz frequencies. Most soft tissues, including the corneal tissues and crystalline lens, exhibit viscoelastic properties characterized by frequency-dependent moduli. Slower relaxation processes have little time to respond to fast mechanical or acoustic modulation, such as GHz acoustic phonons, and thus hardly contribute to the "softness" of the material. As a consequence, modulus tends to increase with frequency. In addition, the propagation of acoustic phonons can be governed by the longitudinal modulus, which is typically much higher than the Young's or shear modulus owing to the incompressibility (*i.e.* Poisson's ratio ~0.5) of water. The effects, finite relaxation time and low compressibility, can provide qualitative explanation for the observed large difference in modulus between the Brillouin and standard mechanical tests.

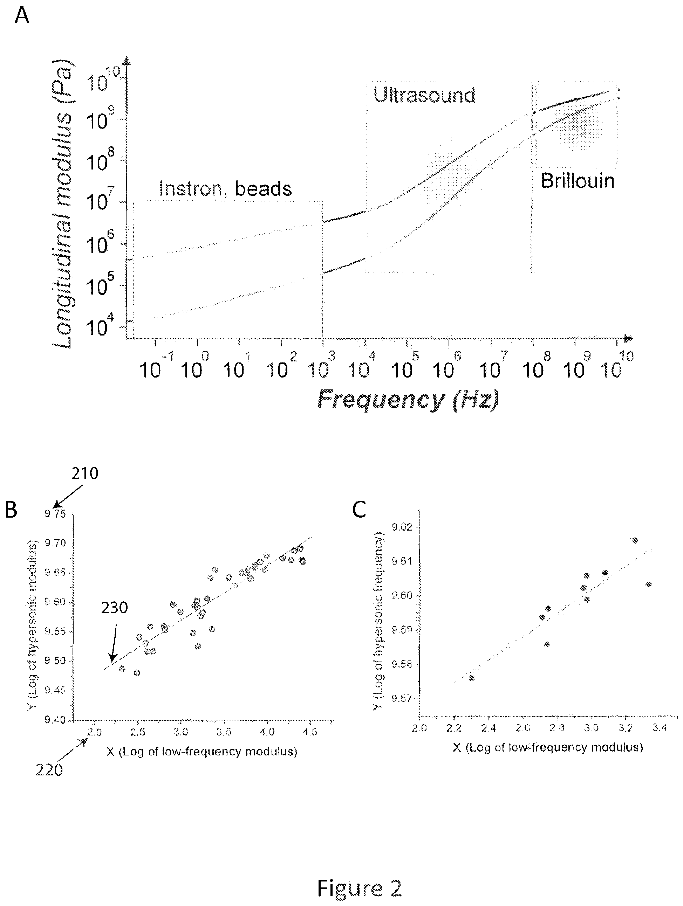

Figure 2A illustrates a graph which associated with the frequency-dependent longitudinal modulus of typical viscoelastic biological samples. Although it is possible that most physiological processes occur in the time scale that corresponds to the low frequency range, e.g. about 0.01-100 Hz, the hypersonic Brillouin measurement can provide an effective way to probe the low-frequency mechanical properties. According to an exemplary embodiment of the present disclosure, a correlation can be established between the Brillouin measurement and conventional mechanical tests, which can represent a calibration to use Brillouin technology for mechanical characterizations.

A correlation experiment has been performed with bovine and porcine lenses. Fresh porcine and bovine lenses have been cut at various ages (from 1 to 18 months) into small pieces of the size our mechanical equipment could handle. The mean Brillouin modulus was calculated from the 3D measurement of Brillouin spectrum and the estimated density and refractive index. Figure 2B-2C shows graphs of exemplary measurement results between the conventional Instron stress-strain test (x-axis) and the Brillouin measurement (y-axis). For example, Brillouin-measured elasticity 210 is much higher than the traditional DC elasticity 220. This is because at higher frequency of strain-perturbation, all relaxation effects are not sampled thus yielding a stiffer than expected reading. Nevertheless, Figure 2B-2C demonstrates an evident relationship between Brillouin measurement and another technique, which indicates that the Brillouin signature provides information about the elasticity of lenticular tissue.

A comparison to Young's modulus measured by a conventional stress-strain test indicates a correlation 230 between Brillouin (*M'*) and quasi-static modulus (*G'*) for both porcine and bovine tissues. High correlation (*R*>0.9) was obtained in curve fit to a log-log linear relationship: log(*M*') = *a* log(*G'*) + *b,* where the fitting parameters were *a*=0.093 and *b*=9.29 for porcine tissues and *a*=0.034 and *b*=9.50 for bovine tissues.

Therefore, the exemplary measurement of the spectral characteristics of the Brillouin scattered light provides the information about the biomechanical properties of the ocular tissue. The useful information obtained by the Brillouin measurement using the exemplary embodiments of the present disclosure includes but is not limited to the acoustic speed, acoustic attenuation coefficient, Brillouin elastic modulus, Brillouin viscous modulus, and electrostriction coefficient. As described further below, by scanning the focus within the tissue different spatial locations can be probed, which can provide the information in a spatially resolved manner. This spatial information can in turn be useful to evaluate for the diagnosis of the mechanical integrity or health of the ocular tissue.

The index of refraction and acoustic speed of a given material are generally dependent on the local temperature and pressure. This dependence may be obtained for the analysis of inflammatory or pathologic states in the eye via the measurement of the temperature or ph-value in the aqueous and vitreous humors. The magnitude of the Brillouin scattered radiation is related to the coupling of acoustic and optical energy inside the sample, which is related to the material properties, such as the electrostriction coefficient.

For example, performing Brillouin microscopy in the eye may not be trivial. Brillouin spectroscopy in the human eye can generally use a low power of illumination and a careful design of the focusing microscope. The potential risks associated with light exposure for Brillouin analysis should be considered. Maximum permissible exposure (MPE) is defined as the highest power or energy density that can be admitted to the eye without causing a biohazard. MPE corresponds to 10% of the dose that has a 50% chance of creating damage in worst-case scenario conditions. In the Brillouin scanner, the laser light can be focused in the cornea and diffused onto the retina. According to the International Commission on Non-Ionizing Radiation Protection (ICNIRP), for cw sources in the wavelength region 400-1050 nm the exposure limit for cornea-lens thermal safety is 4 W/cm2 ; i.e. MPE = 32 mW in a 1-mm-diameter zone (0.79 mm² in area)[19]. The 1-mm aperture for irradiance averaging can be based on the thermal modeling[20], which shows that the temperature rise is independent of the beam size up to 1 mm due to rapid thermal conduction (~0.5 W/m/°C) in the cornea and lens.

As for the retina, the dominant mechanism of retinal damage can be thermal for an exposure time (T) longer than 0.25 s. Considering the distance 17 mm between the lens and retina and α=0.2, i.e. NA=0.1, the beam size on the retina is >3 mm. Applying the same thermal limit of 4 W/cm² , we calculate MPE = ~300 mW. According to the guidelines from American National Standard Institute, the exposure limit for the retinal thermal hazard has been expressed as 1.8x 10⁻³ C_{A} C_{E} T^{-0.25} [W/cm² ], where CA =1-1.5 (for λ=400-800 nm), CE =267 (for a=0.2; i.e. NA=0.1), and the aperture size of 7 mm (area of 0.38 cm²)[21]. The MPE is calculated to be 183 mW for T=1 s and 66 mW for T=60 s, which is consistent with the above analysis. Taken together, the Brillouin scan in the cornea and lens does not pose risk to the human eye, if laser light power is below 3 mW.

The low illumination power places a requirement on the sensitivity of the instrument capturing Brillouin scattered light. Moreover, the back-reflected components from optical components inside the microscope as well as back -scattered signals from the eye due to the difference in index of refraction between the various regions of the eye, in most situations and for most instruments would overshadow Brillouin scattering signal. This can place a requirement on the extinction of the instrument capturing Brillouin scattered light. Additionally, Brillouin signatures from ocular tissue will range between 9 and 15 GHz for the lens and 7.5 to 10 GHz for the cornea; thus, the spectral resolution of the instrument can be extremely high (e.g., less than about 1 GHz) in order to detect relevant changes in the biomechanics of the eye.

According to certain exemplary embodiment of the present disclosure, these simultaneous can be utilized for an exemplary system/arrangement to detect weak Brillouin scattering from the eye, spectrally analyze it and reconstruct a two-dimensional or three-dimensional, microscopic, mapping of the mechanical properties of the eye.

For example, a spectrometer with high resolution, high sensitivity and high extinction in the spectral analysis can be used with the exemplary system/apparatus. Previously, Fabry-Perot (FP) interferometry has been used for the spectral analysis of Brillouin signal in both scanning and angle-dispersive configuration. Both of these methods are generally slow, because of their intrinsically limited throughput. In particular, the resolution performance of spectrally dispersive elements are characterized by a parameter known as finesse which can be thought as how many spectral components can be resolved. However, the maximum amount of light that is forwarded to the detector in a Fabry-Perot interferometer is likely inversely proportional to its finesse; if n is the finesse of the Fabry- Perot interferometer, maximum 1/n of the input light can be sent to the detecting device. This tradeoff between achievable spectral resolution and throughput of the instrument is a fundamental limit of prior art which does not include other practical loss mechanisms.

The 1/n throughput limit was overcome in the prior art by a fully parallel- detection spectroscopic approach that uses a diffractive tilted etalon, called virtually-imaged phased array (VIPA) [9] in combination with an array-type detector such as a CCD camera. VIPA's spectral selection is given by the interference of multiple reflections at two optical flats yielding equivalent performances to FP in terms of resolution. However, the coating of the first surface is totally reflective with a narrow AR window to allow all the light to enter the interferometer. Besides minimizing losses, this conventional design avoids all the light being transmitted to the detector. As a consequence, with respect to an equivalent Fabry- Perot spectrometer, the signal strength is improved by a factor n equal to the finesse of the interferometer.

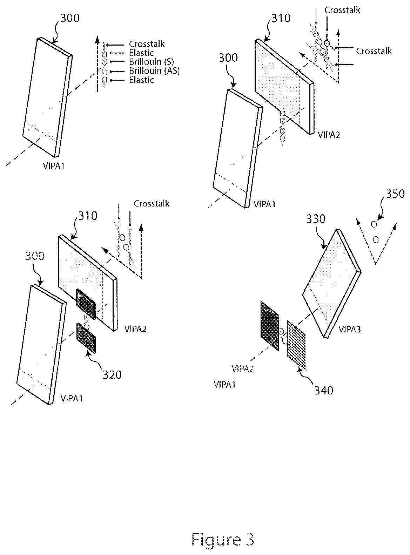

Thus, the spectral unit according to the exemplary embodiment of the present disclosure can include a spectrometer employing at least one virtually imaged phased array (VIPA) etalon 300 as shown in Figure 3A. The V1PA etalon 300 generally disperses the spectrum of input light into different angles or spatial points. However, a VIPA spectrometer has typically a limited extinction of <30 dB (1 over a thousand) and, as a result, it works effectively only in very specific circumstances, i.e. for nearly transparent samples and in the absence of optical back-reflections. In a turbid sample, such as cataractous or damaged eyes, elastic (Rayleigh) scattering can be several orders of magnitude stronger than Brillouin scattering and is separated by only a few GHz from Brillouin signal. In addition, to maximize Brillouin signal collection epi-detection can be used, which increases the back- reflected incident laser component. For this reason, this exemplary embodiment can implement additional spectral selection. Possible variations can include diffraction gratings, fiber Bragg gratings, notch filters based on narrow absorption line of gas cells. Thus, it is possible to provide certain exemplary techniques to increase spectral selection, i.e. multiple VIPA cascading and VIPA apodization. A combination of these techniques can be also implemented.

For example, cascading two or more VIPAs ca be done to increase contrast without hurting significantly the sensitivity of the spectrometer. A single VIPA etalon 300 generally produces spectral dispersion along one spatial direction, parallel to its coating direction, while leaving unchanged the optical beam propagation in the direction perpendicular to its coating direction. Multiple single VIPA etalons can be cascaded in such a way that each VIPA's orientation matches the spectral dispersion axis from the previous stage interferometer. Figure 3 illustrates a set of configurations facilitating a cross-axis cascading. The VIPA 300 in the first exemplary stage of Figure 3 is aligned along the vertical direction and the spectral pattern is dispersed vertically. When the sample is not transparent or when there are strong optical reflections, the elastic scattering component increases dramatically. If the ratio between elastic scattering (dark-green circles) and Brillouin scattering (light-green circles) exceeds the spectral extinction of the spectrometer, a crosstalk signal appears along the spectral axis (green line). This "stray light" can easily overwhelm the weak Brillouin signal.

In a two-stage VIPA, the second etalon 310 can be placed orthogonally to the first one 300. The spectral pattern exiting the first stage can enter the second etalon through the input window. Both etalons disperse light, in orthogonal directions, so the overall spectral axis of the two-stage device lies along a diagonal direction, at 135° from the horizontal axis if the etalons have identical dispersive power. The second etalon 310 can separate Brillouin signal from crosstalk because, although spatially overlapped after the first stage, their frequencies at each spatial location are different. Thus, after the second stage, while the Brillouin spectrum lies on a diagonal axis, crosstalk components due to the limited extinctions of the etalons can be separated and mostly confined to the horizontal and vertical axis.

Besides spatial separation of signal and stray light, the exemplary two-stage spectrometer also facilitates the selective spectral filtering. According to the invention, an appropriate aperture mask 320 is placed at the focal plane of the first VIPA 300, where a highly resolved spectral pattern is formed. Examples of the mask 320 are a slit or a rectangular aperture. This mask allows unwanted spectral components to be blocked and only the desired portion of the spectrum to pass to the second VIPA 310. For a preferred performance, it is possible to maintain only two Brillouin peaks (Stokes and anti-Stokes from two adjacent orders) and have a vertical mask cut off all elastic scattering peaks. This greatly reduces crosstalk in the second-stage VIPA 310 and assist in avoiding a saturation of the pixels in a CCD camera placed afterward, which are illuminated by strong unfiltered elastic scattering light.

This cross-axis cascade can be extended to a third stage. In a three-VIPA spectrometer, a third VIPA 330 is oriented perpendicular to the spectral axis of the preceding two stages, so that the Brillouin spectrum can enter through the input window of the VIPA 330. A second mask 340 can be employed to further reduce crosstalk. Due to the combined dispersion of the three etalons, the overall spectral axis can be further rotated, e.g., at about 170° if all the etalons have the same dispersive power.

Following the same cascading principle, a multiple VIPA interferometer of N stages can be provided. The k-th VIPA can be oriented at an appropriate angle to accept the spectrum dispersed through the preceding *k*-l stages. The building block of each stage is modular, comprised of a cylindrical lens Cₖ, an etalon VIPAₖ , a spherical Fourier transform lens S_{kf} with focal length *f*ₖ, a mask and a spherical relay lens S_{k,k+1} of focal length*f*_{k,k+1}.

In the first stage, the VIPA is oriented along the direction v1 at an angle θ₁ with respect to the horizontal axis (θ₁ = 90° in our experiment), and its spectral dispersion direction, d1, can be parallel to vl with *ψ₁* = *θ₁.* In the double-VIPA interferometer, the second etalon can be aligned along v2 at an angle *θ₂* = *ψ₁* ± *π*/*2*, perpendicular to the spectral direction of the first stage d1 (θ₂ = 180° in our experiment). After the two etalons, the spectrum can emerge at an angle *ψ₂* along spectral dispersion direction, d2. In a three-stage interferometer, the third VIPA must be oriented perpendicular to d2, at an angle *θ₃* = *ψ₂* ± *π*/*2.* This exemplary arrangement can result in a final dispersion direction s3 at an angle *ψ₃.*

For each stage, the exemplary dispersion angle, *ϕₖ,* imposed on a beam of wavelength *λₖ* by the *k^{th}* VIPA interferometer was previously derived in both plane-wave and paraxial approximations. The focal *length fₖ* of the spherical lens, S_{kf}, after the VIPA determines the linear dispersion power, *sₖ,* of the *k^{th}* stage: *sₖ* = *ϕ_{k *} fₖ.* Since telescopes are used to link two subsequent VIPA stages, the overall linear dispersion also depends on the magnifications introduced by such optical systems. For example, each *k^{th}* stage can introduce a magnification *Mₖ* = *fₖ*/*fₖ₋₁* on the spectral pattern obtained by the previous *k-1* stages, so that the effective linear dispersion, *s'ₖ*, due to the *k^{th}* stage is given by: *s'ₖ* = *s_{k *} M_{N *} M_{N-1 *} ... _{*} M*_{*k*+1}*.* Therefore, along the overall spectral axis, the total linear dispersion, *S_{N},* of an N-stage multiple VIPA interferometer is calculated to be: ${S}_{N} = sqrt \left({\sum }_{1}^{N} s {′}_{k}^{2}\right)$, which suggests a theoretical improvement in spectral resolution. When all the spectral dispersions are equal, i.e. *s'*₁ = *s'₂* = ··· = *s'_{N}* ≡ *s*, the total dispersion becomes ${S}_{N} = \sqrt{N} ⋅ s$.

Knowing the spectral dispersion and the optical magnification introduced by each stage, the overall dispersion axis can be computed and/or determined. It can be shown: ${ψ}_{k + 1} - {ψ}_{k} = {tan}^{- 1} \left(s{′}_{k + 1}/{S}_{k}\right) \to {tan}^{- 1} \left(1/\sqrt{k}\right) ;$ (7) ${θ}_{k + 1} - {θ}_{k} = {tan}^{- 1} \left(s{′}_{k}/{S}_{k - 1}\right) \to {tan}^{- 1} \left(1/\sqrt{k - 1}\right) .$ (8)

Here, the expressions marked with arrows apply in the case equal dispersion and unit magnification for all stages.

In terms of extinction, a single VIPA spectrometer has extinction, C proportional to its finesse squared: *C*~4*F*²/*π*², for an input beam with Airy profile. After *N* VIPA etalons of equal finesse *F*, the spectral extinction or contrast improves, in principle, to: *C*~(4*F*²/*π*²)*^{N}.*

It is possible to compare the extinction performance of single-stage, two-stage and three-stage VIPA spectrometers by coupling the single mode laser light directly into the spectrometer and placing a CCD camera in the focal planes of *S_{1f}, S_{2f}*, and *S_{3f}*, respectively To overcome the limited dynamic range of the CCD, it is possible to record the spectrum at various optical power levels, with calibrated neutral density (ND) filters of optical densities in the range from 0 to 7. Subsequently, the full dynamic range spectrum was reconstructed by rescaling the recorded raw spectra according to the respective attenuation levels. The single- stage VIPA shows an extinction level of about 30 dB over a wide frequency range between 5 and 25 GHz. The extinction can be improved to 55 dB with two stages and to nearly 80 dB with three VIPA etalons in the middle of the frequency range. It might be possible to improve the extinction up to 90 dB by minimization of aberrations in the optical system and improvements of beam shape or profile.

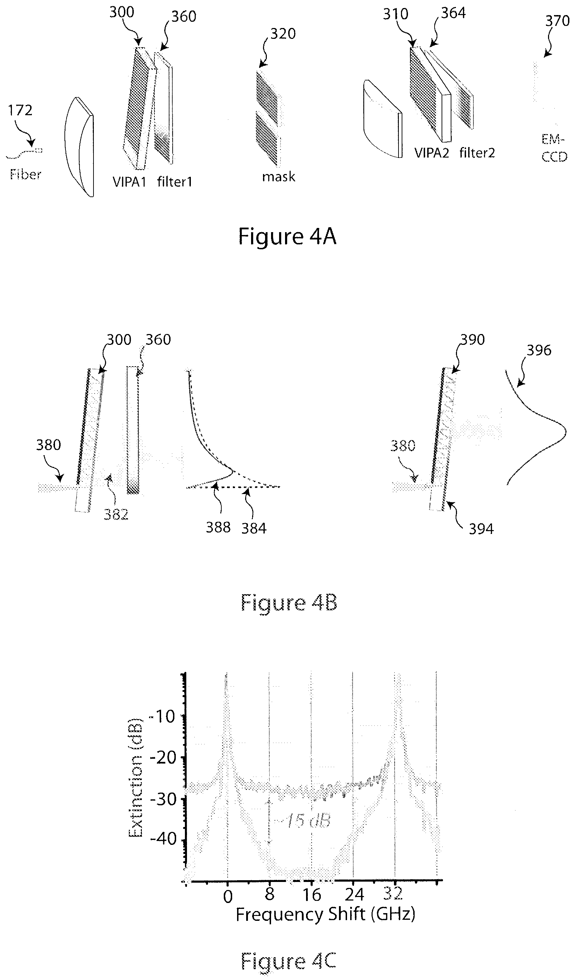

Besides the cross-axis cascading, another approach to improve the extinction ration of a VIPA etalon is to make the intensity profile of the VIPA output close to a Gaussian shape, a technique known as apodization. Figure 4A shows an exemplary configuration according to an exemplary embodiment of a spectrometer using apodized VIPA etalons of the present disclosure. According to the invention, a spatial filter 360 with a spatially varying transmission profile cab be used just after the first VIPA etalon 300. The filter 360 can convert an otherwise exponential beam profile to a rounded shape, such as, e.g., a truncated Gaussian profile. After the second VIPA etalon 310, another linear variable filter 365 can be utilized. The spectrally dispersed output can then be imaged on to a detection unit 370. The detection unit 370 can be a two-dimensional camera based on charge-coupled device (CCD) or a linear detector array.

Figure 4B shows a set of exploded views and usages of the apodization filter 360 according to an exemplary embodiments of the present disclosure. For example, an input light 380 can enter the etalon 300, and can be converted to an output beam 380 consisting of a phased array. For example, in the absence of the filter 360, the intensity of this output beam 380 has an exponential profile 384. The transmission profile of the filter 360 can have a linear, exponential, or more complex nonlinear gradient along the length. The variable filter 360 with an appropriately designed transmission profile can convert the exponential profile 384 to a more round, Gaussian-like profile 388. Passing through a Fourier-transform lens in front of the detection unit 370, the rounded profile can produce a significantly less crosstalk or higher extinction ratio than conventional VIPA etalons. This effect can be demonstrated using a gradient density filter at VIPA output (as shown in the graph of Figure 4C), and improved the extinction by 15 dB with 2 dB excess loss.

In another exemplary variant of the apodization, the VIPA etalon 390 can be made with a gradient coating on the exit surface 394, such that its reflectivity and transmission is varied spatially, producing a rounded, Gaussian-like intensity profile 396, as shown in a righthand portion of Figure 4B. For example, with a single VIPA with its reflectivity of the coating 394 is linearly varied from 99.9% to 90%, an extinction ratio of approximately 59 dB can be achieved in principle assuming a constant phase profile. Gradient reflectivity generally results in a spatially varying phase profile. A linear phase chirp likely may not affect the extinction much and can be compensated for by employing a wedge. With 15 evaporated coating layers, the reflectivity can be made to vary from 92% to 98.5 over 15 mm along the surface 394 can yield a lambda/100 deviation from a wedge. With more layers, a higher reflectivity gradient from 92% to 99.5% can be achieved at the expense of increased nonlinear phase variation of about lambda/40. Extinction improvements of about 20 to 30 dB with minimal losses are predicted by numerical simulation.

At the output of the spectrometer light can be collected by a spatially-resolved photo-detector. Given the paucity of the expected Brillouin photons, high sensitivity solutions are required. Several technologies are known in the art for this purpose, CCD cameras, array of photodiodes, array of photomultiplier tubes of small active area, CMOS, or quadrant photodetectors. For some of these solutions, tight control or tracking over the long-term frequency drift of the laser and/or the spectrometer may be necessary.

For exemplary situations in which high spectral resolution and contrast are needed a scanning filter such as a Fabry-Perot interferometer can be used. The Fabry-Perot scanning interferometer can have a free spectral range of about 50 GHz, and finesse of about 1000; it can operate in single-pass configuration or in multipass, fixed or tandem, to enhance contrast. Alternatively, a fixed filter with a bandpass, notch, or edge type may be used, instead of scanning filter, to measure the magnitude of certain frequency component. In such case, the optical frequency of the pump wave can be stabilized or locked with respect to the fixed filter. The exemplary apparatus can employ different detection techniques, including interferometric heterodyne detection, or different illumination sources such as tunable lasers, or double-frequency pump sources.

In another exemplary embodiment of the present disclosure, other spectral modalities can be added in parallel to Brillouin spectroscopy such as Raman and fluorescence spectroscopy. This would allow rapid and complete fingerprint of ocular properties including, mechanical, chemical and functional information. Since fluorescence, Raman and Brillouin spectrum are in different region of the electromagnetic spectrum, after the collection optics, scattered and fluorescent light can undergo a coarse spectral dispersion to separate the fluorescence and Raman information about the sample under test. Preferred solutions for such coarse spectral dispersion are gratings, dichroic mirrors, interferometric bandpass filters. These exemplary solutions facilitate delivering Raman, Brillouin and fluorescent light to the same CCD camera. Different spatial regions of the CCD camera will map to a different spectral scale to allow simultaneous characterization in three different spectral regimes. Alternatively, the different signatures can be directed to different detecting devices operated in synch This multi-modality can be advantageous because the processes involved sample independent and diverse characteristics of a given material thus yielding mechanical as well as optical and chemical information about the analyzed sample.

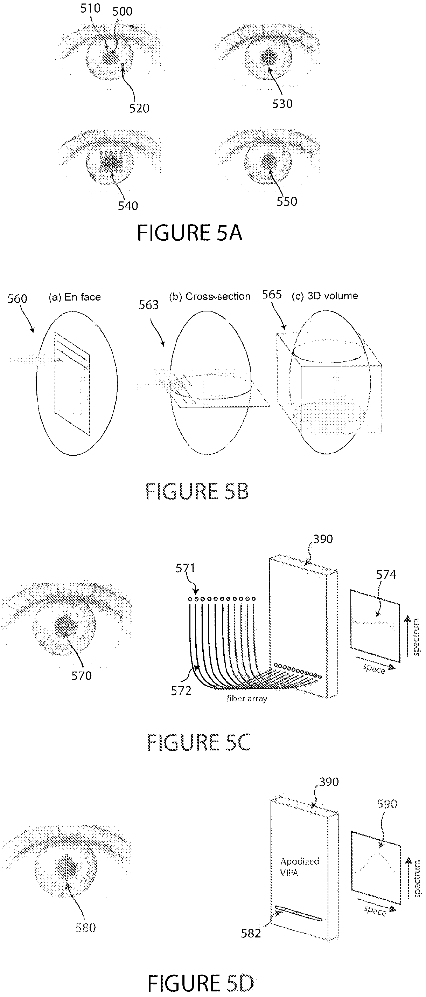

To facilitate Brillouin imaging, the focused light can be scanned over the sample (eye). Figure 5A shows various examples illustrating how the focus 132 is scanned over the eye 120 to obtain the biomechanical information at multiple locations in ocular tissues and thereby to obtain Brillouin images. Various scan types can include axial line scan, lateral line scan, raster area scan, three-dimensional scan, and random sampling scan. In one example, the focus of the probe light can be positioned at a center of the cornea or the lens. As shown in Figure 5A, when such on-axis focus 500 is scanned along the depth coordinate (i.e. Z axis), an axial profile of the biomechanical information, or Brillouin axial profile, is obtained. An off-axis axial profile is obtained by using an off-axis focus 510 displaced from the optic axis of the cornea or the crystalline lens. For corneal scan, far-off-axis focus 520 can be used, in which case the iris blocks the probe light from entering the crystalline lens. In another examples, lateral line-scan or 2-dimensional cross-sectional scan can be achieved by moving a focus along a linear trace 530. For example, 2-dimensional en face or 3-dimensional scan can be achieved by moving the focus over an area in the X and Y coordinates. A simple raster scan 540 or hexagonal scan 550 may be used.

To optimize the imaging speed and sampling resolution, additional beam scanning patterns can be evaluated: XY *en face* model 560, XZ modes 563, and XYZ volµmetric imaging 565, as illustrated in Figure 5B.

In this context, a single-stage VIPA spectrometer with apodization reaching ~60dB of extinction, can be configured to interrogate multiple spatial points simultaneously in the ocular tissues, thus greatly reducing the acquisition time of a Brillouin image. Figures 5C and 5D show two examples. In particular, a multiple foci 570 of the probe light can be formed, and the Brillouin scattered light 571 from each focus can be relayed and coupled to the VIPA etalon 390 through free space or a fiber array 572. The spatio-spectral pattern 574 projected on the detection unit can then be processed to provide the biomechanical information about the ocular tissue interrogated.

Another exemplary method to interrogate multiple spatial points can include a use of a line focus 580. The Brillouin scattered light 582 produced from the line focus 580 can be relayed and coupled to the etalon 390. The spatio-spectral pattern 590 projected on the detection unit can be used to generate the information about the ocular tissues.

According to one exemplary embodiment of the present disclosure using which testing was performed, a light source, imaging optics, a spectrometer, and a computer can be utilized. The light sources for the two prototypes are a frequency-doubled diode-pumped Nd- YAG laser emitting a 532-nm wavelength with a line width of 1 MHz and a grating-stabilized single longitudinal mode laser diode emitting at 780 nm with a line width of about 100 MHz. Light can be focused on a sample through a 35 mm or a 1 1 mm focal length lens. In the prototypes, it is possible to use a beam scanner and a motorized translation stage to move the sample. It is possible to use the epi-detection configuration so that scattered light is collected through the same lens. A single mode fiber was used as confocal pinhole.

Brillouin-scattered light from the sample can be coupled into the VIPA spectrometer for high spatial separation of the spectral components in the plane of an Electron-Multiplied CCD camera. The spectrometer can employ a 3 nm bandpass filter to block fluorescence light. The optical design which can be used for the prototype can include a combination of a two-stage VIPA spectrometer and a variable attenuation neutral density filter. The spectrometer features a spectral resolution of about 1 GHz, an extinction ratio of about 75 dB, and a total insertion loss of 7 dB with a finesse of about 40.

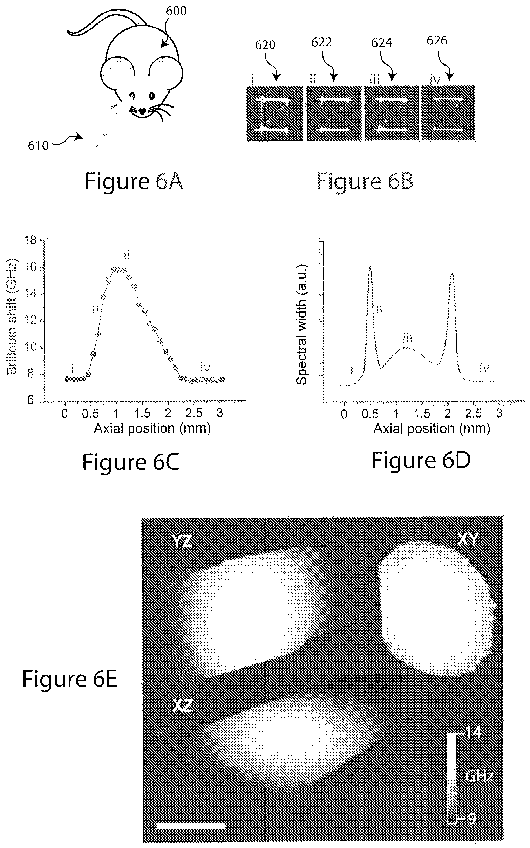

To test the possibility of measuring the lens elasticity *in vivo,* Brillouin imaging was performed on laboratory mice 500 (C57BL/6 strain), as illustrated in Figure 6A. The probe beam 610 was focused into the eye of the mouse under anesthesia. As the animal was moved by a motorized stage, the optical spectrum of scattered light was recorded. Figure 6B shows an illustration of unprocessed data recorded by the camera in the spectrometer at different depths along the ocular optic axis of the lens, featuring the spectral pattern in the anterior cortex 620 (i), lens nucleus 622 (ii), posterior cortex 624 (iii), and vitreous humor 626 (iv). Each spectrum was acquired in 100 ms. Figure 6C shows a plot of the exemplary Brillouin frequency shift measured as a function of depth in the region spanning from the aqueous humor through the lens to the vitreous. Figure 6D illustrates a graph of an exemplary axial profile of the width of the Brillouin spectrum over depth. From these curves, several diagnostic parameters can be derived, such as the peak Brillouin shift at the center of the nucleus, peak Brillouin line width, average frequency shift across the lens, etc.

Figure 6E shows an illustration of exemplary cross-sectional Brillouin elasticity maps, where the color represents the measured Brillouin frequency shift. The image areas are 1.7 x 2 mm² (XY), 1.8 x 3. 1 mm² (YZ), and 2 x 3.5 mm² (XZ), respectively. With a sampling interval of 100 µπ 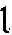 , it took ~2 s to scan each axial line (20 pixels), ~50 s for a cross-sectional area (20x25 pixels), and ~20 min over an entire 3D volume. These exemplary images visualize the gradient of modulus increasing from the outer cortex to inner nucleus.

Using *in vivo* Brillouin microscopy, the natural age dependence of the lens modulus has been investigated. The peak Brillouin shift observed at the center of the lens nucleus in a mouse at 18 month old was 16 GHz, whereas the shift in a younger mouse at 1 month old was about 1 1.5 GHz. The study was extended to 12 mice of different ages to find an evident trend of age-related stiffening. Next, one mouse was imaged every week for two months and obtained consistent age-related data. The exemplary results indicate a quantitative (linear-log) relationship between the hypersonic elastic modulus and the animal age. This exemplary result indicates the first *in vivo* data evidencing an age-related stiffening of lenses in mice.

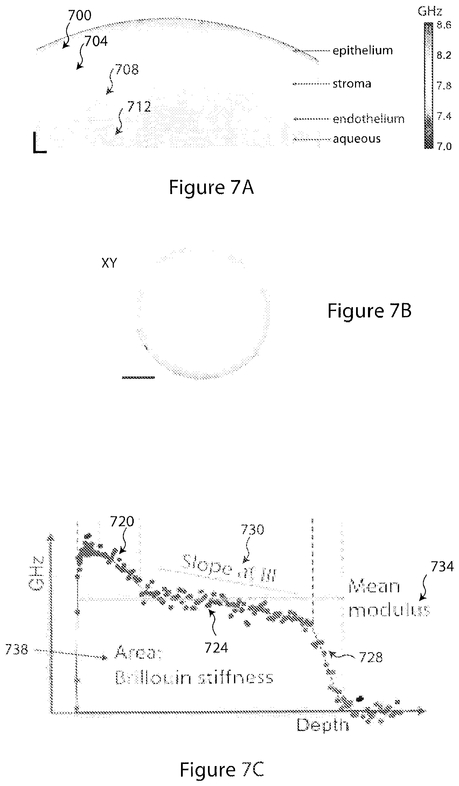

The feasibility of measuring the corneal elasticity was also tested. Brillouin imaging was performed on bovine eyes *ex vivo.* Figure 7A depicts an exemplary Brillouin image of the anterior segment of the bovine eye. The transverse and axial resolution of the probe beam was about 1 and 5 µm, respectively. The Brillouin frequency shift is encoded in color. The depth-resolved cross-sectional image (XZ) indicates that the Brillouin frequency decreases gradually from the epithelium 700 through the stroma 704 to the endothelium 708. The Brillouin frequency of the aqueous humor 712 is consistent with the exemplary shown in Fig. 6C. The variation of the elastic modulus along the depth seems to correlate with the morphological structure of the cornea. The Brillouin modulus did not significantly vary along transverse dimensions in normal cornea. Figure 7B shows an *en face* (XY) image of the cornea optically sectioned at a flat plane.

Figure 7C shows a graph of an exemplary laterally averaged axial profile (along the X-axis) obtained from the central 0.5-mm wide portion of the cross-sectional image in Figure 7A. Several features were observed, such as a steep slope 720 of the Brillouin frequency over depth in the epithelium and the anterior part of stroma, a mild and apparent decreasing slope 724 in the central part, and a rapid decreasing slope 728 in the innermost layers of the stroma toward the endothelium.

Certain exemplary biomechanical diagnostic parameters can be extracted from Brillouin images. For example, the slope 730, defined as the rate of change of elastic modulus across depth; the mean modulus 734, defined as the spatial average of the elastic modulus; and the Brillouin stiffness 738 defined as the area under the Brillouin profile curve. These exemplary parameters can be defined for the whole cornea, or limited to specific regions (e.g. anterior, central and posterior).

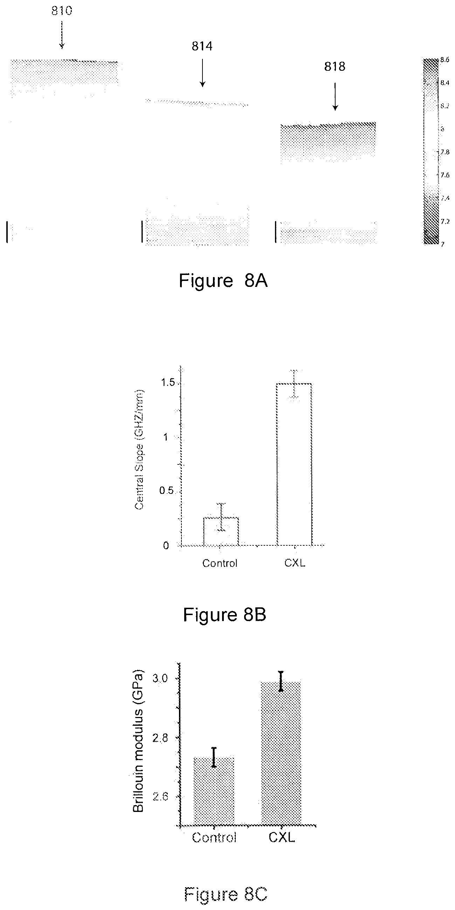

On bovine corneas, the possible use of the exemplary Brillouin technique has been reviewed as a monitoring tool for corneal procedures. It was tested if Brillouin biomechanical imaging is sensitive to corneal stiffness changes induced by therapeutic procedures known as corneal collagen crosslinking (CXL). CXL is a technique that promotes the formation of covalent bonds between collagen fibers inside corneal stroma through a photosensitizing agent and light irradiation. Enhancing the amount of crosslinks between collagen fibers leads to a suffer corneal stroma. The CXL procedure was performed on bovine corneal samples. A photosensitizer (Riboflavin) was diffused into the stroma of the cornea after removal of the epithelium and was activated by illuminating blue light. Figure 8A shows a set of exemplary Brillouin cross-sectional images of bovine cornea samples in three different states: intact 810 , after removal of epithelium 814, and after the crosslinking procedure 81 . It is apparent that the crosslinking procedure greatly has enhanced Brillouin modulus in the stroma. The observed shrinking of tissue after crosslinking is known in the art.

Using Brillouin parameters, it is possible to quantify the effect of CXL procedure. Using analogous procedure to the one described previously, we obtained corneal axial profiles before and after the CXL. In particular, CXL resulted in a large increase (6-fold) in the downward slope of Brillouin frequency over depth in the stromal region. Figure 8B shows a graph with an increase of the central slope (absolute value) for control versus treated samples (N=4). The difference was statistically significant with a p-value of <0.0001 in unpaired two-tailed t-test. Figure 8C shows a graph with a statistically significant increase of the mean Brillouin modulus averaged along the depth profile. The increase in the treated tissues was about 10%.

The relevance of the extracted biomechanical information for clinical purposes can be important. While it may not be very difficult to find studies that relate mechanical properties to physiological or pathological conditions, the use of the exemplary Brillouin technique and its hypersonic elastic properties and that its sensitivity to measure relevant changes is important, which can be address, by analyzing a human ocular tissue.

As for the crystalline lens, he loss of accommodation power leading to presbyopia likely affects every person beyond the age of 50. Age-related stiffening of the crystalline lens is widely considered to play a primary role in this process. However, data are highly variable in terms of magnitude and rate of elasticity change with age as well as internal regional variation of elastic modulus. The uncertainty in the data has prevented a definitive understanding of the aging lens biomechanics and of the accommodation mechanism thus hindering the progress of lens-based treatments for presbyopia. According to an exemplary embodiment of the present disclosure, the axial profile of 14 fresh human lenses *ex vivo* between 21 and 67 yrs of age has been tested. The exemplary spatially-resolved data clearly indicate that the age-related changes in the spatial distribution of elasticity are responsible for the age-related stiffening linked to presbyopia onset.

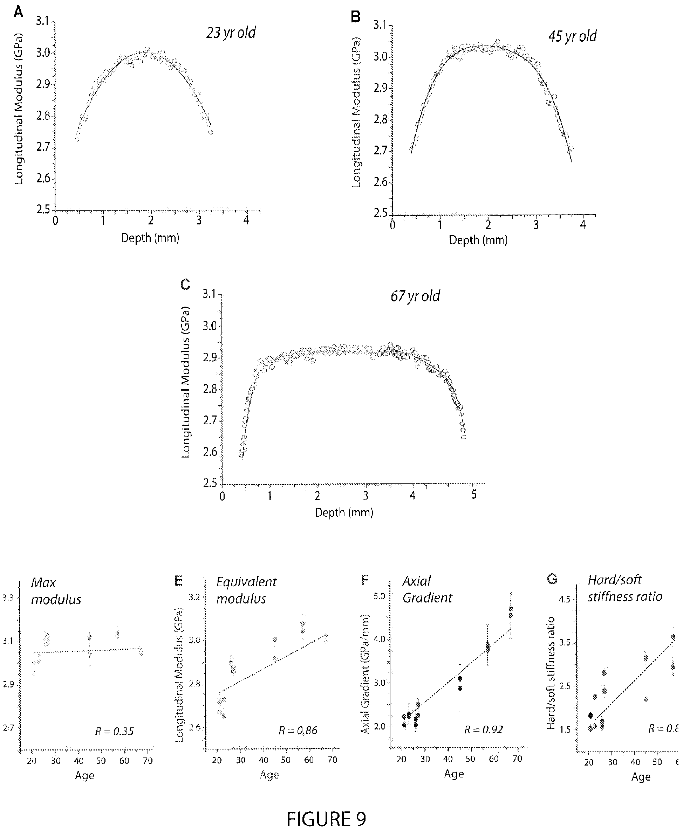

Figures 9A-9C show graphs of the exemplary profiles of the longitudinal elastic modulus of two fresh crystalline lenses (23-, 45-, and 67-yr old, respectively) obtained with Brillouin microscopy. They clearly show that, due to a different elasticity profile, the "equivalent" modulus (e.g., spatial average) or the stiffness (e.g., area under the curve) yield much higher values for the older lens. Figures 9D-9G illustrating associated graphs provide such exemplary observation over the entire age range. From Brillouin scans, e.g., several parameters factoring the spatial distribution of elasticity, namely the equivalent modulus, the spatial elasticity gradient, the "hard-to-soft" stiffness ratio, are highly correlated with age.

As for the cornea, the relevance of Brillouin measurements for keratoconus and corneal ectasia should be understood. Traditional stress-strain tests have indicated that keratoconic corneas have lower mechanical modulus than normal corneas. The significantly altered collagen networks observed in keratoconic corneas could explain the reduced modulus. Keratoconic corneas also have a substantial decrease in the number of crosslinks between collagen fibers[38]. The exemplary Brillouin modulus can be sensitive to such structural changes in corneal collagen network (see Figures 7A-7C). Furthermore, the alterations of collagen network were found to be inhomogeneous and heterogeneous. This can imply that the 3-dimensional analysis by Brillouin microscopy may facilitate a detection of such regional variations. This can represent a feature of Brillouin microscopy.

Exemplary Brillouin axial profiles of exemplary cornea samples have been measured from advanced keratoconus patients and five normal controls. As shown in the exemplary graphs of Figures 1 OA- IOC, the profiles of corneas classified as normal 1010 and keratoconus 1020 can be dramatically different. The negative Brillouin slope of the keratoconic corneas was about 0.94 +/- 0.15 GHz/mm, statistically significantly increased from about 0.36 +/- 0.25 GHz/mm for the controls (unpaired t-test P <0.0025). The mean Brillouin modulus was about 2.58 +/-0.096 GPa for keratoconus corneas and 2.72 +/- 0.06 GPa for normal corneas (e.g., unpaired t-test P <0.05). Exemplary Brillouin data can show a distinction between normal and keratoconus samples, a much bigger separation than what's shown with ORA measurements. This can indicate that the Brillouin parameters, such as slope, modulus and stiffness, can be appropriate metrics sensitive to less dramatic changes expected from earlier stages of keratoconus.

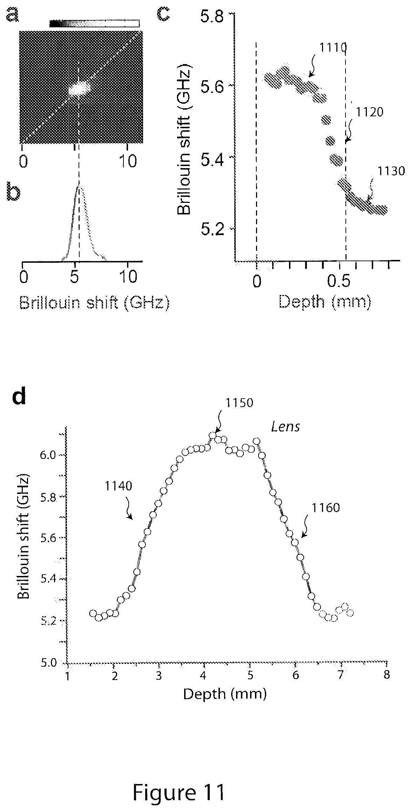

Using the exemplary infrared prototype system, it was possible to obtain an early *in vivo* study of cornea and lens in a human subject. Standard topography and pachymetry did not show any noticeable abnormality before and after Brillouin scans. With low- coherence interferometry, the central corneal thickness was measured to be about 530 µm. For exemplary Brillouin scan of the eye of the volunteer, the beam focus was translated along the optical axis from the cornea to the lens at a speed of 50 um/s (in air), and the Brillouin spectra were acquired with a CCD frame rate of about 2.5 Hz (i.e. integration time of about 0.4 s). Incident light power at the sample was measured to be about 0.7 mW, which is about 30 times lower than the safety limit. Figures 11A-11D shows graphs of exemplary measured axial profiles obtained from the left eye of the subject from both cornea and lens. The exemplary graphs present an average of four such scans, each lasting about 60 s, taken ten minutes apart from each other. In Figure 11C, due to the relatively low confocal resolution (~60 µm in tissue), strong Fresnel reflection from the corneal surface made it difficult to analyze the first 70 µm from the corneal surface. In the anterior portion of the cornea 1110 between about 100 µm and 400 µm, which corresponds to the corneal stroma, the Brillouin shift declined slowly from ~5.6 GHz to ~5.5 GHz; in the posterior region of the cornea 1120, the Brillouin profile showed a steeper decline from about 5.5 GHz to 5.25 GHz in about 150 µm. The aqueous humor 1130 was measured to have a fairly constant Brillouin modulus, slightly higher than the typical Brillouin shift of pure water. As for the crystalline lens, the axial profile of the lens shown in Fig. 11D was obtained with the 35-mm achromatic lens with an axial resolution of about 350 µm. It features a typical bell shape with a slope in the anterior cortex 1140, a plateau at about 6.05 GHz in the lens nucleus 1150, and a decline in the posterior cortex 1160 towards the vitreous humor. A follow-up scan of the same human subject was performed after a month and obtained consistent results, which indicate the repeatability of the technology.

Therefore, it has been confirmed that the features illustrated in animal studies and in human samples *ex vivo* are also measurable *in vivo,* and the instrument is sensitive to detect the elasticity of human cornea and lens.

Finally, the exemplary configuration has been tested beyond the use with the ocular tissue, to demonstrate its applicability and significance with turbid biological tissue, e.g. fresh porcine skin. For example, skin includes two major layers, e.g., epidermis and dermis. The skin tissue sample was cut into thin slices of epidermis and dermis and placed them horizontally on a glass-bottom dish plate with a few saline drops on top to prevent drying. For this measurement, 20 mW of incident light focused onto the tissue through the glass plate was used. Figure 12A shows a graph of a representative Brillouin spectrum from porcine epidermis taken at about 40 µm depth with acquisition time of 300 ms. The spectrum visually confirms the ability of the instrument to suppress the elastic scattering component coming from the turbid tissue. It is possible to effectively reject elastic scattering and obtain Brillouin spectra with SNR greater than one from skin tissue at depths up to 100 microns.

Degradation of skin elasticity is a common problem that can be caused by many factors including age, injuries and diseases. Brillouin confocal microscopy using the apparatus and methods according to the exemplary embodiments of the present disclosure can address skin elasticity below the superficial layer at the microscopic level. With low-power probe beams and rapid spectral readout, the exemplary instrument can serve as a non-invasive non-contact tool to monitor skin elasticity. From a mechanical standpoint, the viable epidermis, below the stratum corneum (~10 microns), can be composed by living cells and is softer than the dermis, mostly made up by collagen and elastin fibers. In agreement with the microstructural composition of the skin layers, exemplary Brillouin spectroscopy, as illustrated in the graphs of Figure 12B, showed a higher frequency shift for the dermal layer (about 8.8±0.1 GHz) with respect to the epidermis (about 7.8±0.1 GHz).

A factor determining skin elasticity can be hydration. Dry skin can have higher Young's modulus of elasticity than hydrated skin by two to three orders of magnitude. Figure 4C shows a graph of the exemplary Brillouin shift of the epidermal layer (about 30 microns below the surface) in hydrated conditions, i.e. after about 10-minute soaking in water, and dry conditions, i.e. after 30 minutes drying in air. The higher water content lowers the Brillouin shift (about 7.6±0.1 GHz) with respect to physiological conditions; in contrast, dry epidermis has significantly higher Brillouin shift (15.4±0.2 GHz).

Regarding Presbyopia, the exemplary Brillouin imaging according to the exemplary embodiments of the present disclosure can facilitate establishing the age- dependent spatially-varying elastic properties most involved in the decline of accommodation power. This can provide mechanistic insights on the accommodation process. This may facilitate a narrow investigation of the underlying molecular processes connected with the accommodation function through the biomechanical information, to address/improve the effectiveness of current procedures for softening refilling the lens, and to design, develop and test other approaches to preserve/restore accommodation inspired by the understanding of lens accommodation biomechanics.

With respect to Keratoconus and Crosslinking the exemplary Brillouin imaging according to the exemplary embodiments of the present disclosure can be used for both early identification of keratoconus and customization of treatment. For example, people suffering from keratoconus can present mechanical abnormalities before clinical symptoms are noted. In addition, the exemplary Brillouin imaging according to the exemplary embodiments of the present disclosure can detect localized weakness of the cornea, thus identifying regions that are more susceptible to the degenerative progression of this disease. This will facilitate extending the practice of crosslinking to early keratoconus individuals. This will also facilitate devising crosslinking procedures that are spatially targeted to the weak corneal regions as well as customized to the specific strengthening need of each patient in terms of photosensitizer and light dose to use. For example, the exemplary Brillouin imaging according to the exemplary embodiments of the present disclosure can be used to obtain a mechanical feedback mechanism that can guide the crosslinking procedures in real time. To this end, the quantitative biomechanical parameters described in this invention are poised to become standardized indexes to evaluate the biomechanical health of the cornea and treatment outcome.

Concerning LASIX, the exemplary Brillouin imaging according to the exemplary embodiments of the present disclosure and its extracted parameters can be used for pre-screening test to judge the compatibility of the patient cornea with LASIK surgery. Individuals with low Brillouin elasticity can be probably counseled to avoid the procedure. For example, once the cornea is mapped out biomechanically, patient currently excluded because of thin corneas or because of too high correction/ablation needed, might be considered safe on the basis of their Brillouin corneal strength. In addition, the ablation patterns can be modified so that the biomechanically strong portions of the cornea are left intact during the procedure. This may yield similar vision correction outcomes, with significantly reduced risk to destabilize the mechanical integrity of the cornea.

Turning to Glaucoma, applanation tonometry is generally used to monitor intraocular pressure for glaucoma screening and management. The test is based on Goldmann equations derived from the Imbirt-Fick law. It is well known in the art that this measurement needs to be corrected for corneal thickness, curvature and elasticity. Correction formulae for thickness and curvature has been described. The exemplary procedure according to the exemplary embodiments of the present disclosure can address a correction of the corneal elasticity component of the intraocular pressure results obtained with standard applanation tonometry. To this end, the protocol calls for previous systematic calibration to be performed combining the exemplary Brillouin microscope, an applanation tonometer and a syringe manometer. As first calibration, using ex vivo eyeballs, the IOP can be manipulated by inserting a syringe manometer into the anterior chamber of an intact eye ex vivo. The IOP measured by applanation tonometry can be compared with the "true" IOP controlled by the syringe manometer while monitoring the corneal elasticity from Brillouin microscopy as well as the corneal thickness and curvature. Several measurements should be done in different conditions of corneal elasticity, including: (1) eyes from different species such as rabbit, pig and cow, human; (2) eyes with stiffened corneas by collagen crosslinking; and (3) eyes with softened corneas by loosening the corneal collagen fibers using various chemicals. This can facilitate deriving a quantitative correction formula for applanation tonometry based on the novel corneal elasticity data from the Brillouin microscopy.

In light of these technological advancement and enabling measurements, the exemplary apparatus and method according to the exemplary embodiments of the present disclosure can facilitate ophthalmic research and patient care since they can provide non-invasive, non-contact and microscopic information on ocular biomechanics *in situ.* The Brillouin ocular analyzer can be proven to be a useful diagnostic tool, facilitating early diagnosis, screening of at-risk patients, monitoring therapeutic responses, developing novel approaches for treatment, and understanding pathogenesis.

The foregoing merely illustrates the principles of the disclosure. Various modifications and alterations to the described embodiments will be apparent to those skilled in the art in view of the teachings herein. Indeed, the arrangements, systems and methods according to the exemplary embodiments of the present disclosure can be used with and/or implement any OCT system, OFDI system, SD-OCT system or other imaging systems, and for example with those described in International Patent Application PCT/US2004/029148, filed September 8, 2004 which published as International Patent Publication No. WO 2005/047813 on May 26, 2005, U.S. Patent Application No. 11/266,779, filed November 2, 2005 which published as U.S. Patent Publication No. 2006/0093276 on May 4, 2006, and U.S. Patent Application No. 10/501,276, filed July 9, 2004 which published as U.S. Patent Publication No. 2005/0018201 on January 27, 2005, and U.S. Patent Publication No. 2002/0122246, published on May 9, 2002. It will thus be appreciated that those skilled in the art will be able to devise numerous systems, arrangements, and procedures which, although not explicitly shown or described herein, embody the principles of the disclosure and can be thus within the scope of the invention, which is defined by the appended claims. It should be understood that the exemplary procedures described herein can be stored on any computer accessible medium, including a hard drive, RAM, ROM, removable disks, CD-ROM, memory sticks, etc., and executed by a processing arrangement and/or computing arrangement which can be and/or include a hardware processors, microprocessor, mini, macro, mainframe, etc., including a plurality and or combination thereof. In addition, certain terms used in the present disclosure, including the specification, drawings and claims thereof, can be used synonymously in certain instances, including, but not limited to, e.g., data and information. It should be understood that, while these words, and/or other words that can be synonymous to one another, can be used synonymously herein, that there can be instances when such words can be intended to not be used synonymously.

## Claims

1. A system for rapid spectroscopic analysis of a biological tissue comprising:
a radiation source (100) configured to provide a first electromagnetic radiation (110) to the biological tissue at an intensity without damaging the biological tissue, wherein the first electromagnetic radiation (110) is configured to interact with an acoustic wave in the biological tissue to produce a second electromagnetic radiation (144) that is different from the first electromagnetic radiation (110);
an imaging system (150) including a spectrometer comprising:
a first stage (300) positioned to receive at least a portion of the second electromagnetic radiation (144),
a second stage (310) positioned to receive at least a portion of the second electromagnetic radiation (144) from the first stage,
and further,
an aperture mask (320) positioned in close proximity to a focal plane between the first and second stages and configured to block at least a portion of an intensity profile of the second electromagnetic radiation (144), the blocked portion including a subset of spectral components of a resolved spectral pattern, and
a first filter (360) positioned between the first stage and the aperture mask and configured to change a shape of the intensity profile of the second electromagnetic radiation (144) as a function of spatial distance transverse to a propagation direction of the second electromagnetic radiation (144).

2. The system of claim 1, wherein each of the stages of the spectrometer comprise a virtually imaged phased array (VIPA) etalon that is configured to disperse the spectrum of the second electromagnetic radiation (144), and the VIPA etalons being cascaded with an orientation of each VIPA etalon corresponding to a spectral dispersion axis associated with an adjacent VIPA etalon.

3. The system of claim 2, wherein the spectrometer further comprises a second filter and a detector, and wherein the second filter is positioned between the second stage and the detector and configured to change a shape of the intensity profile of the second electromagnetic radiation (144) as a function of spatial distance transverse to a propagation direction of the second electromagnetic radiation (144), and optionally or preferably wherein the spectrometer includes a second aperture mask positioned in proximity to a focal plane different from the focal plane between the first and second stages and configured to block a further portion of the intensity profile of the second electromagnetic radiation (144).

4. The system of claim 1, wherein the spectrum is used to determine a frequency difference between the first electromagnetic radiation (110) and the second electromagnetic radiation (144), wherein the frequency difference is associated with a propagation speed of the acoustic wave, and wherein the frequency difference is in a range of 2 to 20 GHz, and optionally or preferably wherein the spectrum is used to determine at least one of maximum, average, or rate of variation of the frequency difference.

5. The system of claim 4, wherein the system is configured to produce at least one image or a spatially-resolved map of the in vivo biological tissue using at least one parameter associated with the frequency difference.

6. The system of claim 1, wherein the system is configured to determine at least one of: a biomechanical property, a stiffness, a cross-linking of the biological tissue, or an intraocular pressure of the biological tissue using the spectrum.

7. A method for rapid spectroscopic analysis of a biological tissue comprising:
receiving a second electromagnetic radiation (144) produced based on an interaction of an acoustic wave in the biological tissue and a first electromagnetic radiation (110) provided to the biological tissue at an intensity that does not damage the biological tissue, wherein the second electromagnetic radiation (144) is different from the first electromagnetic radiation (110);
determining a spectrum of the second electromagnetic radiation (144) using a spectrometer that comprises:
a first stage (300) positioned to receive at least a portion of the second electromagnetic radiation (144),
a second stage (310) positioned to receive at least a portion of the second electromagnetic radiation (144) from the first stage,
and further,
an aperture mask (320) positioned in close proximity to a focal plane between the first and second stages and configured to block at least a portion of an intensity profile of the second electromagnetic radiation (144), the blocked portion including a subset of spectral components of a resolved spectral pattern, and
a first filter (360) positioned between the first stage and the aperture mask and configured to change a shape of the intensity profile of the second electromagnetic radiation (144) as a function of spatial distance transverse to a propagation direction of the second electromagnetic radiation (144).

8. The method of claim 7, wherein each of the stages of the spectrometer comprise a virtually imaged phased array (VIPA) etalon that is configured to disperse the spectrum of the second electromagnetic radiation (144), and the VIPA etalons being cascaded with an orientation of each VIPA etalon corresponding to a spectral dispersion axis associated with an adjacent VIPA etalon.

9. The method of claim 8, wherein the spectrometer further comprises a second filter and a detector, and wherein the second filter is positioned between the second stage and the detector and configured to change a shape of the intensity profile of the second electromagnetic radiation (144) as a function of spatial distance transverse to a propagation direction of the second electromagnetic radiation (144), and optionally or preferably wherein the spectrometer includes a second aperture mask positioned in proximity to a focal plane different from the focal plane between the first and second stages and configured to block a further portion of the intensity profile of the second electromagnetic radiation (144).

10. The method of claim 7, further comprising:
determining a frequency difference between the first electromagnetic radiation (110) and the second electromagnetic radiation (144) using the spectrum of the second electromagnetic radiation (144), wherein the frequency difference is associated with a propagation speed of the acoustic wave, and wherein the frequency difference is in a range of 2 to 20 GHz.

11. The method of claim 10, further comprising:
producing at least one image or a spatially-resolved map of the in vivo biological tissue using the at least one parameter associated with the frequency difference.

12. The method of claim 7, further comprising:
determining at least one of maximum, average, or rate of variation of the frequency difference using the spectrum of the second electromagnetic radiation (144).

13. The method of claim 7, further comprising:
determining at least one of a biomechanical property, a stiffness, a cross-linking of the biological tissue, or an intraocular pressure of the biological tissue using the spectrum.

## Patentansprüche

1. System zur schnellen spektroskopischen Analyse eines biologischen Gewebes, umfassend:
eine Strahlungsquelle (100), die dazu konfiguriert ist, dem biologischen Gewebe eine erste elektromagnetische Strahlung (110) in einer Intensität bereitzustellen, ohne das biologische Gewebe zu schädigen, wobei die erste elektromagnetische Strahlung (110) dazu konfiguriert ist, mit einer akustischen Welle in dem biologischen Gewebe zu interagieren, um eine zweite elektromagnetische Strahlung (144) zu erzeugen, die sich von der ersten elektromagnetischen Strahlung (110) unterscheidet;
ein Bildgebungssystem (150), das ein Spektrometer beinhaltet, das Folgendes umfasst:
e ine erste Stufe (300), die dazu positioniert ist, mindestens einen Abschnitt der zweiten elektromagnetischen Strahlung (144) zu empfangen,
e ine zweite Stufe (310), die dazu positioniert ist, mindestens einen Abschnitt der zweiten elektromagnetischen Strahlung (144) von der ersten Stufe zu empfangen, und ferner
e ine Blendenmaske (320), die in unmittelbarer Nähe einer Fokusebene zwischen der ersten und der zweiten Stufe positioniert und dazu konfiguriert ist, mindestens einen Abschnitt eines Intensitätsprofils der zweiten elektromagnetischen Strahlung (144) zu blockieren, wobei der blockierte Abschnitt eine Teilmenge von Spektralkomponenten eines aufgelösten Spektralmusters beinhaltet, und
e in erstes Filter (360), das zwischen der ersten Stufe und der Blendenmaske positioniert und dazu konfiguriert ist, eine Form des Intensitätsprofils der zweiten elektromagnetischen Strahlung (144) in Abhängigkeit von einer räumlichen Entfernung quer zu einer Ausbreitungsrichtung der zweiten elektromagnetischen Strahlung (144) zu ändern.

2. System nach Anspruch 1, wobei jede der Stufen des Spektrometers ein Etalon mit einem virtuell abgebildeten phasengesteuerten Feld (Virtually Imaged Phased Array) (VIPA-Etalon) umfasst, das dazu konfiguriert ist, das Spektrum der zweiten elektromagnetischen Strahlung (144) zu dispergieren, und die VIPA-Etalons mit einer Ausrichtung jedes VIPA-Etalons kaskadiert sind, die einer Spektraldispersionsachse entspricht, die einem benachbarten VIPA-Etalon zugeordnet ist.

3. System nach Anspruch 2, wobei das Spektrometer ferner ein zweites Filter und einen Detektor umfasst und wobei das zweite Filter zwischen der zweiten Stufe und dem Detektor positioniert und dazu konfiguriert ist, eine Form des Intensitätsprofils der zweiten elektromagnetischen Strahlung (144) in Abhängigkeit von einer räumlichen Entfernung quer zu einer Ausbreitungsrichtung der zweiten elektromagnetischen Strahlung (144) zu ändern, und wobei optional oder vorzugsweise das Spektrometer eine zweite Blendenmaske beinhaltet, die in der Nähe einer Fokusebene positioniert ist, die sich von der Fokusebene zwischen der ersten und der zweiten Stufe unterscheidet, und dazu konfiguriert ist, einen weiteren Abschnitt des Intensitätsprofils der zweiten elektromagnetischen Strahlung (144) zu blockieren.

4. System nach Anspruch 1, wobei das Spektrum verwendet wird, um einen Frequenzunterschied zwischen der ersten elektromagnetischen Strahlung (110) und der zweiten elektromagnetischen Strahlung (144) zu bestimmen, wobei der Frequenzunterschied einer Ausbreitungsgeschwindigkeit der akustischen Welle zugeordnet ist und wobei der Frequenzunterschied in einem Bereich von 2 bis 20 GHz liegt und wobei optional oder vorzugsweise das Spektrum dazu verwendet wird, mindestens eines von maximaler, durchschnittlicher oder Rate der Variation des Frequenzunterschieds zu bestimmen.

5. System nach Anspruch 4, wobei das System dazu konfiguriert ist, mindestens ein Bild oder eine räumlich aufgelöste Karte des biologischen In-vivo-Gewebes unter Verwendung mindestens eines Parameters zu erzeugen, der dem Frequenzunterschied zugeordnet ist.

6. System nach Anspruch 1, wobei das System dazu konfiguriert ist, mindestens eines der Folgenden zu bestimmen: eine biomechanische Eigenschaft, eine Steifigkeit, eine Vernetzung des biologischen Gewebes oder einen intraokularen Druck des biologischen Gewebes unter Verwendung des Spektrums.

7. Verfahren zur schnellen spektroskopischen Analyse eines biologischen Gewebes, umfassend:
Empfangen einer zweiten elektromagnetischen Strahlung (144), die auf Grundlage einer Interaktion einer akustischen Welle in dem biologischen Gewebe und einer ersten elektromagnetischen Strahlung (110) erzeugt wird, die dem biologischen Gewebe in einer Intensität bereitgestellt ist, die das biologische Gewebe nicht schädigt, wobei sich die zweite elektromagnetische Strahlung (144) von der ersten elektromagnetischen Strahlung (110) unterscheidet;
Bestimmen eines Spektrums der zweiten elektromagnetischen Strahlung (144) unter Verwendung eines Spektrometers, das Folgendes umfasst:
e ine erste Stufe (300), die dazu positioniert ist, mindestens einen Abschnitt der zweiten elektromagnetischen Strahlung (144) zu empfangen,
e ine zweite Stufe (310), die dazu positioniert ist, mindestens einen Abschnitt der zweiten elektromagnetischen Strahlung (144) von der ersten Stufe zu empfangen, und ferner
e ine Blendenmaske (320), die in unmittelbarer Nähe einer Fokusebene zwischen der ersten und der zweiten Stufe positioniert und dazu konfiguriert ist, mindestens einen Abschnitt eines Intensitätsprofils der zweiten elektromagnetischen Strahlung (144) zu blockieren, wobei der blockierte Abschnitt eine Teilmenge von Spektralkomponenten eines aufgelösten Spektralmusters beinhaltet, und
e in erstes Filter (360), das zwischen der ersten Stufe und der Blendenmaske positioniert und dazu konfiguriert ist, eine Form des Intensitätsprofils der zweiten elektromagnetischen Strahlung (144) in Abhängigkeit von einer räumlichen Entfernung quer zu einer Ausbreitungsrichtung der zweiten elektromagnetischen Strahlung (144) zu ändern.

8. Verfahren nach Anspruch 7, wobei jede der Stufen des Spektrometers ein Etalon mit einem virtuell abgebildeten phasengesteuerten Feld (VIPA-Etalon) umfasst, das dazu konfiguriert ist, das Spektrum der zweiten elektromagnetischen Strahlung (144) zu dispergieren, und die VIPA-Etalons mit einer Ausrichtung jedes VIPA-Etalons kaskadiert sind, die einer Spektraldispersionsachse entspricht, die einem benachbarten VIPA-Etalon zugeordnet ist.

9. Verfahren nach Anspruch 8, wobei das Spektrometer ferner ein zweites Filter und einen Detektor umfasst und wobei das zweite Filter zwischen der zweiten Stufe und dem Detektor positioniert und dazu konfiguriert ist, eine Form des Intensitätsprofils der zweiten elektromagnetischen Strahlung (144) in Abhängigkeit von einer räumlichen Entfernung quer zu einer Ausbreitungsrichtung der zweiten elektromagnetischen Strahlung (144) zu ändern, und wobei optional oder vorzugsweise das Spektrometer eine zweite Blendenmaske beinhaltet, die in der Nähe einer Fokusebene positioniert ist, die sich von der Fokusebene zwischen der ersten und der zweiten Stufe unterscheidet, und dazu konfiguriert ist, einen weiteren Abschnitt des Intensitätsprofils der zweiten elektromagnetischen Strahlung (144) zu blockieren.

10. Verfahren nach Anspruch 7, ferner umfassend:
Bestimmen eines Frequenzunterschieds zwischen der ersten elektromagnetischen Strahlung (110) und der zweiten elektromagnetischen Strahlung (144) unter Verwendung des Spektrums der zweiten elektromagnetischen Strahlung (144), wobei der Frequenzunterschied einer Ausbreitungsgeschwindigkeit der akustischen Welle zugeordnet ist und wobei der Frequenzunterschied in einem Bereich von 2 bis 20 GHz liegt.

11. Verfahren nach Anspruch 10, ferner umfassend:
Erzeugen mindestens eines Bildes oder einer räumlich aufgelösten Karte des biologischen In-vivo-Gewebes unter Verwendung des mindestens einen Parameters, der dem Frequenzunterschied zugeordnet ist.

12. Verfahren nach Anspruch 7, ferner umfassend:
Bestimmen von mindestens einem von maximaler, durchschnittlicher oder Rate der Variation des Frequenzunterschieds unter Verwendung des Spektrums der zweiten elektromagnetischen Strahlung (144).

13. Verfahren nach Anspruch 7, ferner umfassend:
Bestimmen von mindestens einem von einer biomechanischen Eigenschaft, einer Steifigkeit, einer Vernetzung des biologischen Gewebes oder eines intraokularen Drucks des biologischen Gewebes unter Verwendung des Spektrums.

## Revendications

1. Système pour une analyse spectroscopique rapide d'un tissu biologique comprenant :
une source de rayonnement (100) configurée pour la fourniture d'un premier rayonnement électromagnétique (110) au tissu biologique à une intensité sans endommagement du tissu biologique, dans lequel le premier rayonnement électromagnétique (110) est configuré pour l'interaction avec une onde acoustique dans le tissu biologique pour la production d'un second rayonnement électromagnétique (144) qui est différent du premier rayonnement électromagnétique (110) ;
un système d'imagerie (150) incluant un spectromètre comprenant :
u n premier étage (300) positionné pour la réception d'au moins une portion du second rayonnement électromagnétique (144),
u n second étage (310) positionné pour la réception d'au moins une portion du second rayonnement électromagnétique (144) en provenance du premier étage, et en outre,
u n masque d'ouverture (320) positionné à proximité étroite d'un plan focal entre les premier et second étages et configuré pour le blocage d'au moins une portion d'un profil d'intensité du second rayonnement électromagnétique (144), la portion bloquée incluant un sous-ensemble de composantes spectrales d'un motif spectral résolu, et
u n premier filtre (360) positionné entre le premier étage et le masque d'ouverture et configuré pour la modification d'une forme du profil d'intensité du second rayonnement électromagnétique (144) en fonction d'une distance spatiale transversale à une direction de propagation du second rayonnement électromagnétique (144).

2. Système selon la revendication 1, dans lequel chacun des étages du spectromètre comprend un étalon à réseau en phase virtuellement imagé (VIPA) qui est configuré pour la dispersion du spectre du second rayonnement électromagnétique (144), et les étalons VIPA étant montés en cascade avec une orientation de chaque étalon VIPA correspondant à un axe de dispersion spectrale associé à un étalon VIPA adjacent.

3. Système selon la revendication 2, dans lequel le spectromètre comprend en outre un second filtre et un détecteur, et dans lequel le second filtre est positionné entre le second étage et le détecteur et configuré pour la modification d'une forme du profil d'intensité du second rayonnement électromagnétique (144) en fonction d'une distance spatiale transversale à une direction de propagation du second rayonnement électromagnétique (144), et éventuellement ou de préférence dans lequel le spectromètre inclut un second masque d'ouverture positionné à proximité d'un plan focal différent du plan focal entre les premier et second étages et configuré pour le blocage d'une portion supplémentaire du profil d'intensité du second rayonnement électromagnétique (144).

4. Système selon la revendication 1, dans lequel le spectre est utilisé pour la détermination d'une différence de fréquence entre le premier rayonnement électromagnétique (110) et le second rayonnement électromagnétique (144), dans lequel la différence de fréquence est associée à une vitesse de propagation de l'onde acoustique, et dans lequel la différence de fréquence est dans une plage de 2 à 20 GHz, et éventuellement ou de préférence dans lequel le spectre est utilisé pour la détermination d'au moins un élément parmi un maximum, une moyenne, ou un taux de variation de la différence de fréquence.

5. Système selon la revendication 4, dans lequel le système est configuré pour la production d'au moins une image ou d'une carte résolue spatialement du tissu biologique in vivo en utilisant au moins un paramètre associé à la différence de fréquence.

6. Système selon la revendication 1, dans lequel le système est configuré pour la détermination d'au moins une parmi : une propriété biomécanique, une rigidité, une réticulation du tissu biologique, ou une pression intraoculaire du tissu biologique en utilisant le spectre.

7. Procédé pour une analyse spectroscopique rapide d'un tissu biologique comprenant :
la réception d'un second rayonnement électromagnétique (144) produit sur la base d'une interaction d'une onde acoustique dans le tissu biologique et d'un premier rayonnement électromagnétique (110) fourni au tissu biologique à une intensité qui n'endommage pas le tissu biologique, dans lequel le second rayonnement électromagnétique (144) est différent du premier rayonnement électromagnétique (110) ;
la détermination d'un spectre du second rayonnement électromagnétique (144) en utilisant un spectromètre qui comprend :
un premier étage (300) positionné pour la réception d'au moins une portion du second rayonnement électromagnétique (144),
un second étage (310) positionné pour la réception d'au moins une portion du second rayonnement électromagnétique (144) en provenance du premier étage, et en outre, un masque d'ouverture (320)
positionné à proximité étroite d'un plan focal entre les premier et second étages et configuré pour le blocage d'au moins une portion d'un profil d'intensité du second rayonnement électromagnétique (144), la portion bloquée incluant un sous-ensemble de composantes spectrales d'un motif spectral résolu, et
un premier filtre (360) positionné entre le premier étage et le masque d'ouverture et configuré pour la modification d'une forme du profil d'intensité du second rayonnement électromagnétique (144) en fonction d'une distance spatiale transversale à une direction de propagation du second rayonnement électromagnétique (144).

8. Procédé selon la revendication 7, dans lequel chacun des étages du spectromètre comprend un étalon à réseau en phase virtuellement imagé (VIPA) qui est configuré pour la dispersion du spectre du second rayonnement électromagnétique (144), et les étalons VIPA étant montés en cascade avec une orientation de chaque étalon VIPA correspondant à un axe de dispersion spectrale associé à un étalon VIPA adjacent.

9. Procédé selon la revendication 8, dans lequel le spectromètre comprend en outre un second filtre et un détecteur, et dans lequel le second filtre est positionné entre le second étage et le détecteur et configuré pour la modification d'une forme du profil d'intensité du second rayonnement électromagnétique (144) en fonction d'une distance spatiale transversale à une direction de propagation du second rayonnement électromagnétique (144), et éventuellement ou de préférence dans lequel le spectromètre inclut un second masque d'ouverture positionné à proximité d'un plan focal différent du plan focal entre les premier et second étages et configuré pour le blocage d'une portion supplémentaire du profil d'intensité du second rayonnement électromagnétique (144).

10. Procédé selon la revendication 7, comprenant en outre :
la détermination d'une différence de fréquence entre le premier rayonnement électromagnétique (110) et le second rayonnement électromagnétique (144) en utilisant le spectre du second rayonnement électromagnétique (144), dans lequel la différence de fréquence est associée à une vitesse de propagation de l'onde acoustique, et dans lequel la différence de fréquence est dans une plage de 2 à 20 GHz.

11. Procédé selon la revendication 10, comprenant en outre : la production d'au moins une image ou d'une carte résolue spatialement du tissu biologique in vivo en utilisant l'au moins un paramètre associé à la différence de fréquence.

12. Procédé selon la revendication 7, comprenant en outre :
la détermination d'au moins un élément parmi un maximum, une moyenne, ou un taux de variation de la différence de fréquence en utilisant le spectre du second rayonnement électromagnétique (144).

13. Procédé selon la revendication 7, comprenant en outre :
la détermination d'au moins une parmi une propriété biomécanique, une rigidité, une réticulation du tissu biologique, ou une pression intraoculaire du tissu biologique en utilisant le spectre.
